# EUROPEAN PATENT SPECIFICATION

(11) **EP 2 978 745 B1**
(45) Date of publication and mention of the grant of the patent: **15.03.2017**
(21) Application number: 14712694.0
(22) Date of filing: 27.03.2014
(51) Int. Cl.: C07D 215/26

(54) **PROCESS FOR THE MANUFACTURE OF (R)-5-[2-(5,6-DIETHYLINDAN-2-YLAMINO)-1-HYDROXYETHYL]-8-HYDROXY-(1H)-QUINOLIN-2-ONE**
VERFAHREN ZUR HERSTELLUNG VON (R)-5-[2-(5,6-DIETHYLINDAN-2-YLAMINO)-1-HYDROXYETHYL]-8-HYDROXY-(1H)-QUINOLIN-2-ON
PROCÉDÉ DE PRÉPARATION DE (R)-5-[2-(5,6-DIÉTHYLINDAN-2-YLAMINO)-1-HYDROXYÉTHYL]-8-HYDROXY-(1H)-QUINOLIN-2-ONE

(30) Priority: 27.03.2013 EP 13161410
(43) Date of publication of application: 03.02.2016
(73) Proprietor: Laboratorios Lesvi, S.L., 08970 Sant Joan Despí - Barcelona (ES)
(72) Inventor: DALMASES BARJOAN, Pere, E-08970 Sant Joan Despí-Barcelona (ES); HUGUET CLOTET, Juan, E-08970 Sant Joan Despí-Barcelona (ES); CAPDEVILA URBANEJA, Enric, E-08970 Sant Joan Despí-Barcelona (ES)
(74) Representative: ABG Patentes, S.L.
(86) International application number: PCT/EP2014/056229
(87) International publication number: WO 2014/154841

(56) References cited:
- WO-A1-00/75114
- WO-A1-2004/076422
- WO-A1-2014/044566
- WO-A2-2013/132514
- FRANCOIS BAUR ET AL: "The Identification of Indacaterol as an Ultralong-Acting Inhaled [beta]2-Adrenoceptor Agonist", JOURNAL OF MEDICINAL CHEMISTRY, vol. 53, no. 9, 2010, pages 3675-3684, XP055121338, ISSN: 0022-2623, DOI: 10.1021/jm100068m

## Description

### FIELD OF THE INVENTION

The present invention provides a process for manufacturing (*R*)-5-[2-(5,6-diethyl-indan-2-yl-amino)-1-hydroxyethyl]-8-hydroxy-(1H)-quinolin-2-one, also known as indacaterol, or a pharmaceutically acceptable salt or co-crystal thereof, in high yield and high purity.

### BACKGROUND OF THE INVENTION

Indacaterol, as a chirally pure compound having formula **I,** is an ultra-long-acting β₂-adrenoreceptor agonist, commercialized as its maleate salt under the brand name of Onbrez Breezhaler® for the once daily treatment of chronic obstructive pulmonary disease (COPD), which is one of the major causes of morbidity and mortality in the developed countries despite the many treatment modalities available.

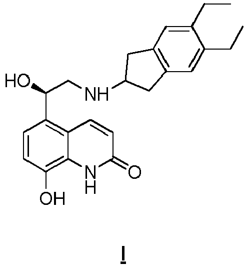

Indacaterol and related compounds were first disclosed in WO00/75114. The synthesis of indacaterol maleate was also described in WO00/75114 as depicted in Scheme 1. The process involved the reaction of an epoxide of formula **V** with an amine intermediate of formula **VI** to give a compound of formula **VII.** The compound of formula **VII,** which was isolated by flash chromatography, was then converted into indacaterol maleate after removing the benzyl group and treating the resulting indacaterol base with maleic acid. Yields and purities were not disclosed in WO00/75114.

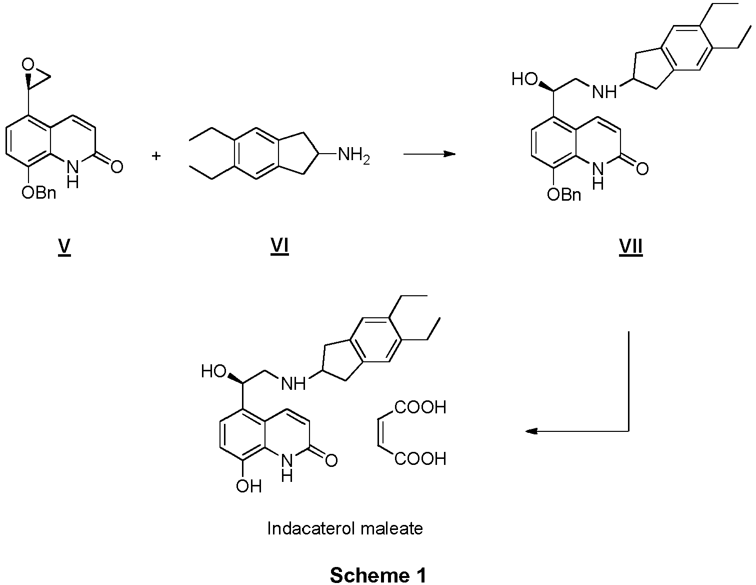

An improved process for the preparation of indacaterol was disclosed in WO2004/76422 and is depicted in Scheme 2, wherein intermediate of formula **VII** was isolated as a benzoate salt (**VII.benzoate**). This route implies several drawbacks. Firstly, the process described in WO2004/76422, provided complex crude containing the regioisomeric byproduct **VIII** and a double alkylated side product of formula **IX,** as depicted in Scheme 2. In addition, a high amount of these impurities, about 20%, were generated. Thus, in order to improve the purity of the crude thus obtained, further steps were carried out. The crude was treated with an acid in the presence of a solvent to yield the corresponding salt of compound **VII.** Several salts were obtained in high purity, however low yields were provided. In addition, other salts such as the hydrochloric acid salt of intermediate **VII** were also obtained in low purity and also in low yield. Thus, the overall yield of the process described in WO2004/76422 resulted in low yields of about 60%.

Secondly, indacaterol directly obtained by the processes described in WO2004/76422 had low purity and had to be further purified in order to be used in the manufacture of pharmaceutical compositions. The purification was carried out by means of chromatographic methods, which are generally expensive, environmentally unfriendly and time consuming, therefore not feasible for industrial application.

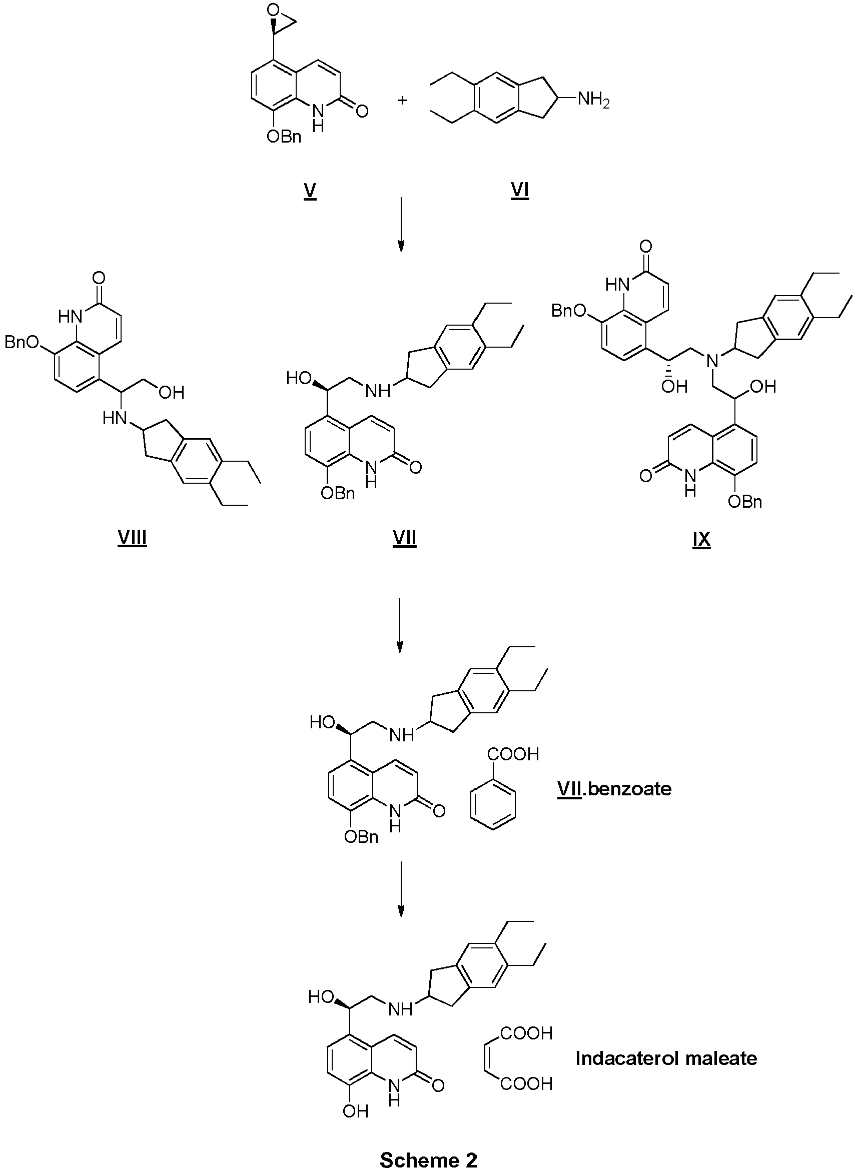

Moreover, document WO2005/123684 discloses a process for preparing intermediate **V** in high enantiomeric purity through the use of a chiral agent and a base. Indacaterol is then prepared following the known process depicted in Scheme 2, which involves the formation of 20% of impurities. Enantiomeric purities are not disclosed.

All the aforementioned disadvantages increase the cost of the final indacaterol process and the pharmaceutical compositions containing it, which has already resulted in expensive medications.

Therefore, there is still a need to develop an industrially feasible process for the manufacture of indacaterol or a pharmaceutically acceptable salt thereof in high purity and high yield.

### BRIEF DESCRIPTION OF THE INVENTION

The present inventors have surprisingly found that the process of the invention provides indacaterol in high chemical and enantiomeric purity and high yield, which makes it appropriate for the preparation of pharmaceutically acceptable salts or co-crystals thereof, particularly indacaterol maleate at industrial scale.

Thus, a first aspect of the present invention provides a process for manufacturing indacaterol of formula **I**, (*R*)-5-[2-(5,6-diethyl-indan-2-yl-amino)-1-hydroxyethyl]-8-hydroxy-(1H)-quinolin-2-one, or a pharmaceutically acceptable salt or co-crystals thereof, which comprises the following steps;
i) reacting compound of formula **II** 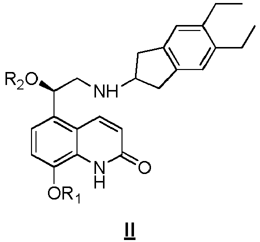 wherein R₁ is an aralkyl group and R₂ is a silyl group, with an acid, HX, selected from the group consisting of hydrochloric acid, hydrobromic acid, phosphoric acid, sulfuric acid, formic acid, acetic acid, dichloroacetic acid, methansulfonic acid, p-toluensulfonic acid and camphorsulfonic acid, in the presence of an organic solvent, to provide a compound of formula **III,** 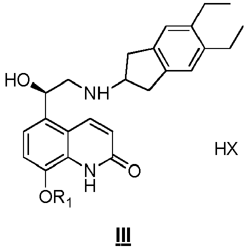 wherein R₁ is defined as above
ii) optionally converting the compound of formula **III** to a compound of formula **III.B** by treatment with a base in the presence of a solvent, wherein the solvent is a mixture of an organic solvent and water, 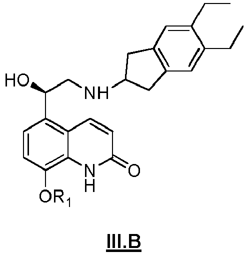
iii) removing the protecting group R₁ from the products resulting from step i) or step ii), when present, by hydrogenation, in the presence of a catalyst and an organic solvent to provide indacaterol of formula **I** 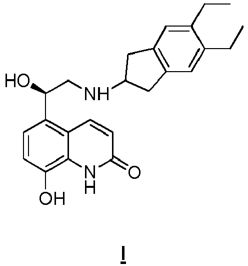
iv) optionally, treating the indacaterol (compound of formula **I**) obtained in step iii) with a pharmaceutically acceptable acid to provide a pharmaceutically acceptable salt or co-crystal of indacaterol

Advantageously, the process of the present invention, which comprises a reduced number of steps, is easily reproducible at industrial scale and leads to indacaterol in high yield. Moreover, significant reduction of byproducts is obtained. In particular, indacaterol is also obtained in high chemical and enantiomeric purity so that the corresponding pharmaceutically acceptable salt, especially its maleate salt, can be prepared in an efficient manner.

Also described is a process for preparing compounds of formula **II** from compound of formula **X** with 2-amino-(5,6-diethyl)-indan or a salt thereof, wherein R₁ and R₂ are as defined hereinbefore and L is a leaving group selected from halogens, alkyl, aryl, aryl alkyl and aralkyl sulfonates.

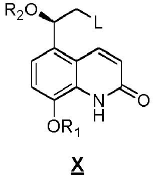

### BRIEF DESCRIPTION OF THE DRAWINGS

Examples of the invention are illustrated with the following drawings:

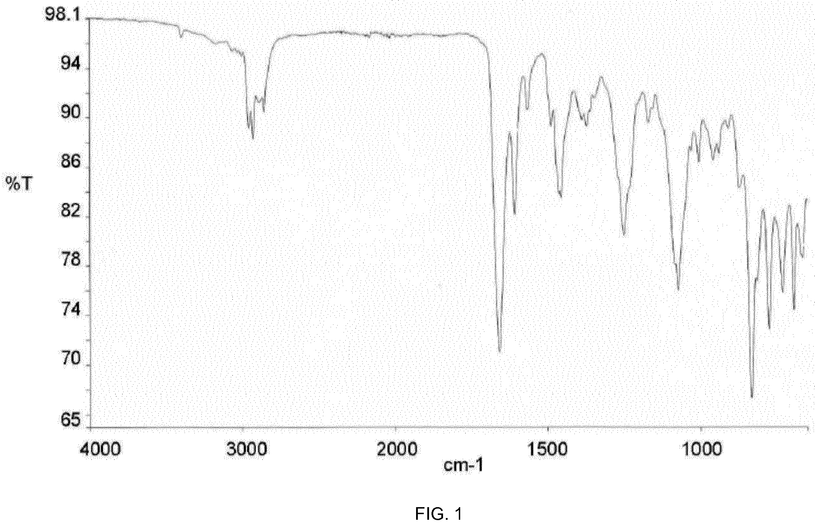

**Figure 1** shows the IR analysis of compound of formula **II;** wherein R₁ is Bn and R₂ is TBDMS; (*R*)-8-(benzyloxy)-5-[1-((*tert*-butyldimethylsilyl)oxy)-2-((5,6-diethyl-2,3-dihydro-1H-inden-2-yl)amino)ethyl]quinolin-2(1H)-one.
**Figure 2** shows the IR analysis of compound of formula **III;** wherein R₁ is Bn and HX is hydrochloric acid.
**Figure 3** shows the DSC analysis of compound of formula **III;** wherein R₁ is Bn and HX is hydrochloric acid.
**Figure 4** shows the PXRD analysis of compound of formula **III;** wherein R₁ is Bn and HX is hydrochloric acid.

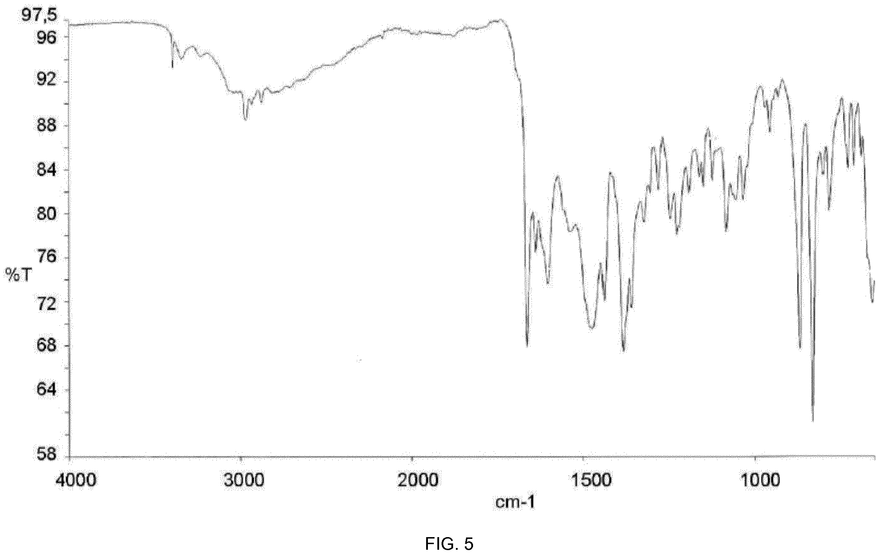

**Figure 5** shows the IR analysis of indacaterol maleate.

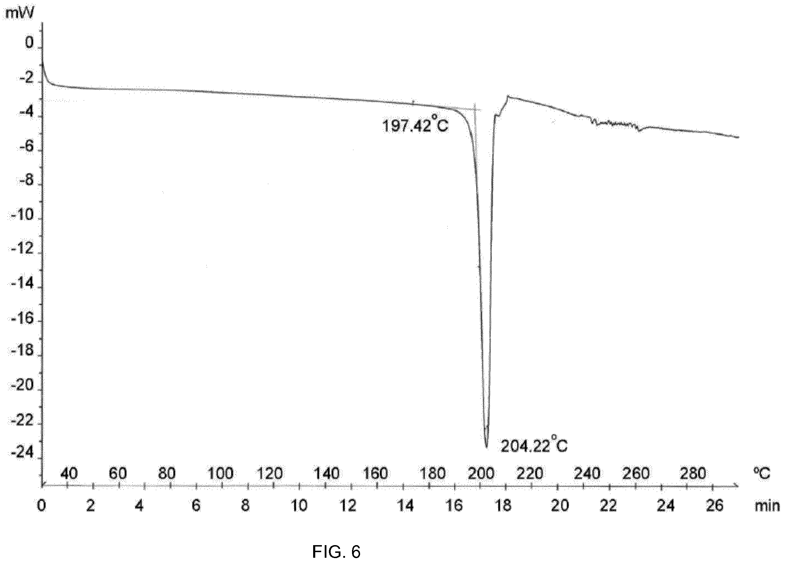

**Figure 6** shows the DSC analysis of indacaterol maleate.

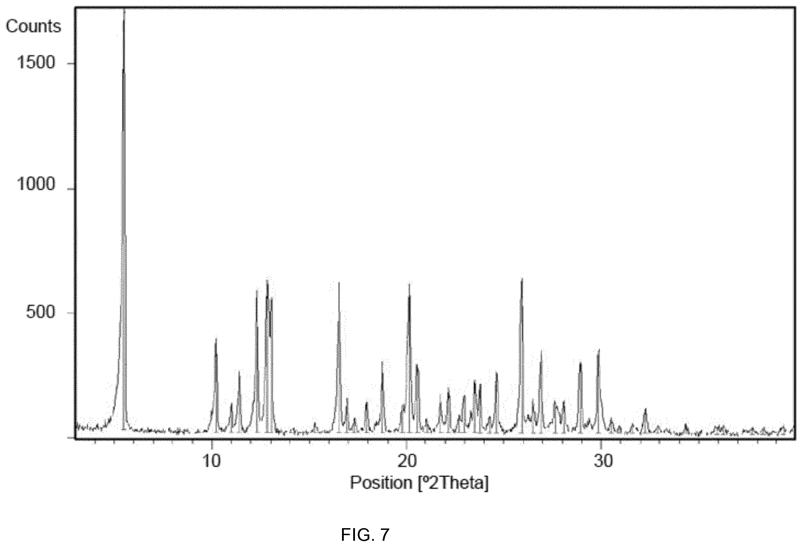

**Figure 7** shows the PXRD analysis of indacaterol maleate.
**Figure 8** shows the IR analysis of compound of formula **III.HA;** wherein R₁ is Bn and HA is L-tartaric acid.
**Figure 9** shows the DSC analysis of compound of formula **III.HA;** wherein R₁ is Bn and HA is L-tartaric acid.
**Figure 10** shows the IR analysis of compound of formula **III.HA;** wherein R₁ is Bn and HA is ditoluyl-L-tartaric acid.
**Figure 11** shows the DSC analysis of compound of formula **III.HA;** wherein R₁ is Bn and HA is ditoluyl-L-tartaric acid.
**Figure 12** shows the IR analysis of compound of formula **III.HA;** wherein R₁ is Bn and HA is ditoluyl-D-tartaric acid.
**Figure 13** shows the DSC analysis of compound of formula **III.HA;** wherein R₁ is Bn and HA is ditoluyl-D-tartaric acid.

### ABBREVIATIONS

Unless indicated otherwise, all indications of percentage are by weight and temperatures are in degrees Celsius. The following abbreviations are used:
- TLC: Thin Layer Chromatography
- HPLC: High Performance Liquid Chromatography
- DMSO: Dimethylsulfoxide
- TMS: Tetramethylsilane
- TBDMS: *tert*-butyl dimethyl silyl
- Bn: Benzyl

### DEFINITIONS

When describing the compounds and methods of the invention, the following terms have the following meanings, unless otherwise indicated.

The term acid refers to a substance that tends to release a proton. The term acid contemplates all inorganic or organic acids. Suitable acids for the present invention are hydrochloric acid, hydrobromic acid, hydroiodic acid, sulfuric acid, phosphoric acid (also known as ortho phosphoric acid), formic acid, acetic acid, dichloroacetic acid, methanesulfonic acid, ethanesulfonic acid, *p*-toluensulfonic acid and camphorsulfonic acid.

As used herein the term **organic solvent** refers to an organic molecule capable of at least partially dissolving another substance (i.e., the solute). Organic solvents may be liquids at room temperature. Examples of organic solvents that may be used for the present invention include, but are not limited to: hydrocarbon solvents (e.g., n-pentane, n-hexane, n-heptane, n-octane, paraffin, cyclohexane, methylcyclohexane, decahydronaphthalene, mineral oil, crude oils, etc.) which also includes aromatic hydrocarbon solvents (e.g., benzene, toluene, o-xylene, m-xylene, and p-xylene), halogenated hydrocarbon solvents (e.g., carbon tetrachloride, 1,2-dichloroethane, dichloromethane, chloroform, etc.), ester solvents (e.g., ethyl formate, methyl acetate, ethyl acetate, isopropyl acetate, butyl acetate, isobutyl acetate, ethyl malonate, etc.), ketone solvents (e.g., acetone, methyl ethyl ketone or 2-butanone, methyl isobutyl ketone, cyclohexanone, cyclopentanone, 3-pentanone, etc.), ether solvents (e.g., diethyl ether, dipropyl ether, diphenyl ether, isopropyl ether, tert-butyl methyl ether, tetrahydrofuran, 1,4-dioxane, etc.), amine solvents (e.g., propyl amine, diethylamine, triethylamine, aniline, pyridine), alcohol solvents (e.g., methanol, ethanol, isopropanol, 1-propanol, 2-methyl-1-propanol, 1-butanol, 2-butanol, 1-pentanol, 3-methyl-1-butanol, tert-butanol, 1-octanol, benzyl alcohol, phenol, trifluoroethanol, glycerol, ethylene glycol, propylene glycol, m-cresol, etc.), acid solvents (e.g., acetic acid, hexanoic acid, etc.), carbon disulfide, nitrobenzene, N,N-dimethylformamide, N,N,-dimethylacetamide, dimethyl sulfoxide, N-methyl-2-pyrrolidone, acetonitrile, silicone solvents (e.g., silicone oils, polysiloxanes, cyclosilicones). In some embodiments, the organic solvent may be formed by the combination of two or more organic solvents.

The term **polar solvent** as used herein means a solvent that tends to interact with other compounds or itself through acid-base interactions, hydrogen bonding, dipole-dipole interactions, or by dipole-induced dipole interactions. Examples of polar solvents are dichloromethane, tetrahydrofuran, ester solvents (e.g., ethyl formate, methyl acetate, ethyl acetate, ethyl malonate, etc.), ketone solvents (e.g., acetone, methyl ethyl ketone or 2-butanone, cyclohexanone, cyclopentanone, 3-pentanone, etc.), amine solvents (e.g., propyl amine, diethylamine, triethylamine, aniline, pyridine), alcohol solvents (e.g., methanol, ethanol, isopropanol, 1-propanol, 1-butanol, 1-octanol, benzyl alcohol, phenol, trifluoroethanol, glycerol, ethylene glycol, propylene glycol, m-cresol, etc.), acid solvents (e.g., acetic acid, hexanoic acid, etc.), carbon disulfide, nitrobenzene, N,N-dimethylformamide, N,N,-dimethylacetamide, dimethyl sulfoxide, N-methyl-2-pyrrolidone, acetonitrile, and silicone solvents (e.g., silicone oils, polysiloxanes, cyclosilicones).

The term **aprotic solvent** as used herein means any molecular solvent which cannot donate H⁺. Examples of aprotic solvents that may be used for the present invention include, but are not limited to: tetrahydrofuran, 2-methyltetrahydrofuran, toluene, methylcyclohexane, acetonitrile, N,N-dimethylformamide, N,N,-dimethylacetamide, dimethyl sulfoxide, N-methyl-2-pyrrolidone, methyl ethyl ketone or 2-butanone, and methyl isobutyl ketone.

The term **alcohol** refers to a hydrocarbon derivative in which one or more hydrogen atoms have been replaced by an -OH group, known as hydroxyl group. Suitable alcohols for the present invention include linear, cyclic or branched C₁-C₆ alkyl alcohols and any mixtures thereof. It also includes commercially available alcohols. Examples of alcohols are methanol, ethanol, isopropanol, 1-propanol and 1-butanol.

The term **inorganic base** as used herein refers to a substance that tends to accept a proton. It contains a metal cation and does not contain an organic moiety, as compared to an organic base, which is a substance that contains an organic moiety. Suitable inorganic bases for the present invention are hydroxides, carbonates and bicarbonates of calcium, sodium, magnesium, potassium, lithium and caesium.

As used herein, the term **tertiary amine** refers to an amine compound containing three alkyl groups. Examples of tertiary amines include triethylamine, diisopropylethylamine and the like.

As used herein, the term **one-pot process** refers to a process comprising simultaneously or successively adding all reactants into a reactor to have them react together, in which no separation and/or purification of the intermediate state is required before the final product is produced.

The term **conventional purification techniques** as used herein refers to the process wherein a product can be obtained in high purity, which can be carried out on an industrial scale such as solvent extraction, filtration, distillation, slurring, washing, phase separation, evaporation, centrifugation, isolation or crystallization.

As used herein, the term, **solvent extraction** refers to the process of separating components of a mixture by using a solvent which possesses greater affinity for one component, and may therefore separate said one component from at least a second component which is less miscible than said one component with said solvent.

The term **filtration** refers to the act of removing solid particles greater than a predetermined size from a feed comprising a mixture of solid particles and liquid. The expression filtrate refers to the mixture less the solid particles removed by the filtration process. It will be appreciated that this mixture may contain solid particles smaller than the predetermined particle size. The expression "filter cake" refers to residual solid material remaining on a feed side of a filtration element.

As used herein, the term **slurring** refers to any process which employs a solvent to wash or disperse a crude product.

As used herein, the term **washing** refers to the process of purifying a solid mass (e.g., crystals) by passing a liquid over and/or through the solid mass, as to remove soluble matter. The process includes passing a solvent, such as distilled water, over and/or through a precipitate obtained from filtering, decanting, or a combination thereof. For example, in one embodiment of the invention, washing includes contacting solids with solvent or solvent mixture, vigorously stirring (e.g., for two hours), and filtering. The solvent can be water, can be an aqueous solvent system, or can be an organic solvent system. Additionally, the washing can be carried out with the solvent having any suitable temperature. For example, the washing can be carried out with the solvent having a temperature between about 0 °C and about 100 °C.

The term **phase separation** refers to a solution or mixture having at least two physically distinct regions.

The term **evaporation** refers to the change in state of solvent from liquid to gas and removal of that gas from the reactor. Generally gas is removed by vacuum applied across the membrane. Various solvents may be evaporated during the synthetic route disclosed herein. As known to those of skill in the art, each solvent may have a different evaporation time and/or temperature.

The term **crystallization** refers to any method known to a person skilled in the art such as crystallization from single solvent or combination of solvents by dissolving the compound, optionally at elevated temperature, and precipitating or allowing to precipitate (crystallize) the compound by cooling the solution, allowing it to cool, removing the solvent from the solution, or any combination thereof. It further includes methods such as those comprising the use of a solvent (for dissolving the compound)/antisolvent (for precipitating the compound) or precipitation.

The term **purification** and/or **purifying** as used herein refer to the process wherein a purified drug substance can be obtained, preferably having a purity greater than 80%, more preferably greater than 85%, more preferably greater than 90%, more preferably greater than 95%, more preferably greater than 99%, more preferably greater than 99.5%, even more preferably greater than 99.9%. The term "industrial purification" refers to purifications, which can be carried out on an industrial scale such as solvent extraction, filtration, slurring, washing, phase separation, evaporation, centrifugation or crystallization.

The term **pure indacaterol** as used herein refers to indacaterol of a purity obtained by HPLC of at least 98% (w/w), preferably of at least 99% (w/w), most preferably of at least 99.5% (w/w).

The term enantiomerically pure indacaterol refers to an enantiomeric purity of the desired R-configuration obtained by chiral HPLC of at least 97% (w/w), preferably of at least 98% (w/w), more preferably of at least 99% (w/w), more preferably of at least 99.5% (w/w), more preferably of at least 99.9% (w/w), even more preferably of at least 99.99% (w/w), even more preferably of 100% (w/w).

The term **pharmaceutically acceptable salt** refers to a salt prepared from a base or acid or their respectives conjugated acids or bases, which is acceptable for administration to a patient, such as a mammal. Such salts can be derived from pharmaceutically acceptable inorganic or organic bases and from pharmaceutically acceptable inorganic or organic acids. Salts derived from pharmaceutically acceptable acids include, but are not limited to, acetic, benzenesulfonic, benzoic, camphosulfonic, citric, ethanesulfonic, fumaric, gluconic, glutamic, hydrobromic, hydrochloric, lactic, maleic, malic, mandelic, methanesulfonic, mucic, nitric, pantothenic, phosphoric, succinic, sulfuric, tartaric, p-toluenesulfonic, xinafoic (1-hydroxy-2-naphthoic acid), 1,5 - naphthalene disulfonic, cinnamic, and the like. Salts derived from pharmaceutically acceptable bases include alkali metal (e.g. sodium or potassium) and alkali earth metal (e.g. calcium or magnesium) hydroxides and organic bases, such as alkyl amines, arylalkyl amines and heterocyclic amines. Preferably, the pharmaceutically acceptable salt refers to a salt with a pharmaceutically acceptable acid. More preferably, the pharmaceutically acceptable acid is maleic acid, acetic acid and 1-hydroxy-2-naphthoic acid.

The term **co-crystal** refers to a unique crystalline structure consisting of the parent compound (indacaterol) and a pharmaceutically acceptable acid, such as those that have been defined above, bounded by weak interactions and/or non-covalent interactions such as hydrogen bond, ionic interactions, van der Waals interactions and Π-Π interactions,wherein the difference of pKa between the pKa of the base (indacaterol) and the pKa of the pharmaceutically acceptable acid is of at least below 3. More preferably, the difference of pKa between the pKa of the base (indacaterol) and the pKa of the pharmaceutically acceptable acid is of at least below 0.

### DETAILED DESCRIPTION OF THE INVENTION

The present inventors have surprisingly found that the process of the invention provides indacaterol in high chemical and enantiomeric purity and high yield, which makes it appropriate for the preparation of pharmaceutically acceptable salts thereof such as indacaterol maleate at industrial scale.

Thus, a first aspect of the present invention provides a process for manufacturing indacaterol, (*R*)-5-[2-(5,6-diethyl-indan-2-yl-amino)-1-hydroxyethyl]-8-hydroxy-(1H)-quinolin-2-one, or a pharmaceutically acceptable salt or co-crystal thereof, comprising the steps of:
i) reacting compound of formula **II** 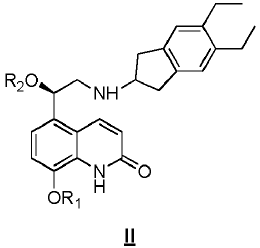 with an acid, HX, in the presence of an organic solvent, wherein R₁, R₂ and HX are defined as above, to provide a compound of formula **III**, 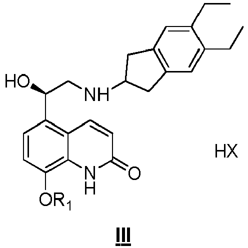 wherein R₁ is defined as above
ii) optionally converting compound of formula **III** to a compound of formula **III.B** by treatment with a base in the presence of a solvent, wherein the solvent is a mixture of an organic solvent and water, 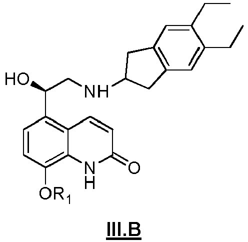
iii) removing the protecting group R₁ from the products resulting from step i) or step ii), when present, by hydrogenation, in the presence of a catalyst and an organic solvent to provide indacaterol of formula **I** 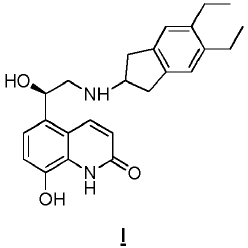
iv) optionally, treating the indacaterol (compound of formula **I**) obtained in step iii) with a pharmaceutically acceptable acid to provide a pharmaceutically acceptable salt or co-crystal of indacaterol

Preferably, in step i) of the process of the present invention, the molar ratio of the compound of Formula **II** to the acid of formula HX is form 1:1 to 1:15.

Advantageously, step i) of the present invention comprises a one-pot process wherein the R₂ hydroxyl protecting group is removed with an acid HX in the presence of an
organic solvent, preferably wherein the molar ratio of the compound of Formula **II** to the acid of formula HX is form 1:1 to 1:15, and an acid addition salt of the corresponding compound of Formula **III** is provided. This, results in a straightforward and reproducible process, which reduces the number of steps for manufacturing indacaterol and decreases the amount of impurities of the final indacaterol.

R₁ and R₂ defined in the compounds of formula **II,** and R₁ defined in the compounds of formula **III,III.HA** and **III.B** are hydroxyl protecting groups. In the compounds of formula **II,** R₁ and R₂ are different.

As used herein, **alkyl** means straight-chain or branched hydrocarbon chain radical containing no insaturation having from 1 to 10 carbon atoms, represented as C₁-C₁₀ alkyl. Such alkyl groups may be selected from methyl, ethyl, n-propyl, isopropyl, n-butyl, isobutyl, sec-butyl, tert-butyl, straight- or branched-pentyl, straight- or branched-hexyl, straight- or branched-heptyl, straight- or branched-nonyl or straight- or branched-decyl. Preferably the alkyl group is C₁-C₄ alkyl.

**Alkoxy** means straight chain or branched radical of the formula -OR, wherein R is an alkyl radical as defined above. Alkoxy may be C₁-C₁₀ alkoxy. Examples of alkoxy groups are methoxy, ethoxy, n-propoxy, isopropoxy, n-butoxy, isobutoxy, sec-butoxy, tert-butoxy or straight- or branched-pentoxy, -hexyloxy, -heptyloxy, -octyloxy, -nonyloxy or -decyloxy. Preferably the alkoxy group is C₁-C₄ alkoxy.

**Alkenyl** means straight chain or branched hydrocarbon chain radical containing one or more double bonds and having 2 to 10 carbon atoms, represented as C₂-C₁₀ alkenyl. Such alkenyl groups may be selected from vinyl, 1-propenyl, 2-propenyl, 1-butenyl, isobutenyl, or straight- or branched-pentenyl, -hexenyl, -heptenyl, -octenyl, -nonenyl or -decenyl. Preferably, the alkenyl is C₂-C₄ alkenyl.

**Aryl** means an aromatic hydrocarbon radical having 6 to 10 carbon atoms such as phenyl or naphthyl. The aryl radical may be optionally substituted by one or more substituents such as OH, SH, halo, alkyl, phenyl, alkoxy, haloalkyl, nitro, cyano, acyl as defined herein.

**Cycloalkyl** means a 3- to 10-membered cyclic radical which is saturated or partially saturated, consisting of carbon and hydrogen atoms any of which can be substituted by one, two or more C₁-C₄ alkyl groups, particularly methyl groups. Examples of cycloalkyl are cyclopropyl, cyclobutyl, cyclopentyl, cyclohexyl or cycloheptyl, any of which can be substituted by one, two or more C₁-C₄ alkyl groups, particularly methyl groups. Preferably, the cycloalkyl is C₃-C₆ cycloalkyl.

**Benzocycloalkyl** means a cycloalkyl as previously defined, which includes one of the C₃-C₁₀ cycloalkyl groups mentioned hereinbefore, fused to a benzene ring. Preferably, the benzocycloalkyl is a benzo-C₅-C₆ cycloalkyl; more preferably, the benzocycloalkyl is benzocyclohexyl (tetrahydronaphthyl).

**Cycloalkylalkyl** means a C₁-C₁₀ alkyl as defined above substituted with a C₃-C₁₀ cycloalkyl as defined above, particularly one of the C₁-C₄ alkyl groups, substituted by one of the C₃-C₁₀ cycloalkyl groups mentioned hereinbefore. Preferably the cycloalkylalkyl is C₃-C₆ cycloalkyl-C₁-C₄alkyl.

**Aralkyl** means C₁-C₁₀ alkyl as defined above substituted with C₆-C₁₀ aryl as defined above. Preferably, the aralkyl group is a C₁-C₄alkyl group, substituted by a C₆-C₁₀ aryl group such as, phenyl, tolyl, xylyl and naphthyl. The C₆-C₁₀ aryl groups may also be substituted by one or more substituents on the ring. Suitable substituents are selected from alkyl, alkoxy, halogen, as defined herein and nitro. The preferred substituents are alkyl and alkoxy groups. Preferred aralkyl groups are 4-methylbenzyl, 2-methylbenzyl, 4-methoxybenzyl or *p*-methoxybenzyl, 2-methoxybenzyl, 2,4-dimethoxybenzyl, 3,4-dimethoxybenzyl, 2,6-dimethoxybenzyl, 4-nitrobenzyl, 2-nitrobenzyl or o-nitrobenzyl, 2,4-dinitrobenzyl, 4-chlorobenzyl and 2-chlorobenzyl. More preferably, the aralkyl is benzyl or *p*-methoxybenzyl. Still more preferably, the aralkyl is benzyl.

**Heteroaralkyl** means straight-chain or branched aralkyl as defined above, which may include one of the C₆-C₁₀ aryl-C₁-C₁₀ alkyl groups mentioned hereinbefore, substituted by one or more heterocyclic groups. Preferably, the aralkyl group is C₆-C₁₀ aryl-C₁-C₄ alkyl substituted by one or more heterocyclic groups.

**Halo** and **halogen** as used herein refer to an atom selected from fluorine, chlorine, bromine and iodine.

**Haloalkyl** means straight-chain or branched alkyl as defined above, such as C₁-C₁₀ alkyl mentioned hereinbefore, substituted by one or more, one, two or three, halogen atoms, preferably fluorine and/or chlorine atoms. Preferably, the haloalkyl is C₁-C₄ alkyl substituted by one, two or three fluorine and/or chlorine atoms.

**Acyl** means C₁-C₆ alkylcarbonyl or C₆-C₁₀ arylcarbonyl, wherein alkyl and aryl are as defined above. The preferred acyl group is a C₆-C₁₀ arylcarbonyl. Cycled C₆-C₁₀ arylcarbonyl may have one or more substituents selected from alkyl, alkoxy, halogen, previously defined and nitro. The preferred substituent is the nitro group. Preferred C₆-C₁₀ arylcarbonyl groups are 4-methylbenzoyl, 2-methylbenzoyl, 4-methoxybenzoyl or p-methoxybenzoyl, 2-methoxybenzoyl, 2,4-dimethoxybenzoyl, 3,4-dimethoxybenzoyl, 2,6-dimethoxybenzoyl, 4-nitrobenzoyl, 2-nitrobenzoyl or o-nitrobenzoyl, 2,4-dinitrobenzoyl, 4-chlorobenzoyl, 2-chlorobenzoyl, 4-bromobenzoyl and the like. Preferably, the C₆-C₁₀ arylcarbonyl group is 2,4-dinitrobenzoyl. Suitable C₁-C₆ alkylcarbonyl groups are selected from acetyl, propionyl, isobutyryl, butyryl, valeroyl and pivaloyl. The preferred acyl group is 2,4-dinitrobenzoyl.

The term **silyl** means a silyl group substituted with at least one C₁-C₁₀ alkyl group as defined above, such as trimethylsilyl, triethylsilyl, dimethylisopropylsilyl, diethylisopropylsilyl, tert-butyldimethylsilyl, triisopropylsilyl and the like. Preferably, the silyl group is tert-butyldimethylsilyl or triethylsilyl. Still more preferably, the silyl group is tert-butyldimethylsilyl.

In the compounds of formula **II** and **III** of the process of the invention, the R₁ hydroxyl protecting group is an aralkyl group. Preferably, the R₁ is a C₁-C₄ alkyl group, substituted by a C₆-C₁₀ aryl group. Preferably, R₁ is selected from the group consisiting of 4-methylbenzyl, 2-methylbenzyl, 4-methoxybenzyl or p-methoxybenzyl, 2-methoxybenzyl, 2,4-dimethoxybenzyl, 3,4-dimethoxybenzyl, 2,6-dimethoxybenzyl, 4-nitrobenzyl, 2-nitrobenzyl or o-nitrobenzyl, 2,4-dinitrobenzyl, 4-chlorobenzyl and 2-chlorobenzyl. More preferably, R₁ is benzyl or *p*-methoxybenzyl. Even more preferably R₁ is benzyl.

In the compounds of formula **II** of the process of the invention, the R₂ hydroxyl protecting group is a silyl group. Preferably, R₂ is selected from the group consisiting of trimethylsilyl, triethylsilyl, dimethylisopropylsilyl, diethylisopropylsilyl, tert-butyldimethylsilyl, triisopropylsilyl. More preferably, R₂ is selected from the group consisiting of tert-butyldimethylsilyl and triethylsilyl. Even more preferably R₂ is tert-butyldimethylsilyl.

In a particular embodiment, R₁ and R₂ of compound of formula **II** are respectively benzyl and tert-butyldimethylsilyl. Thus, R₁ of compound of formula **III** is benzyl.

The acid of Formula HX used in step i) according to the first aspect of the present invention is preferably hydrochloric acid, which may be present either as gas or as an aqueous solution or generated in situ, for example from an alkylsilyl halogenide in the presence of a protic solvent.
The molar ratio of the compound of formula **II** to the acid of Formula HX is from 1:1 to 1:15. More preferably, the molar ratio is from 1:1 to 1:10. Most preferably, the molar ratio of the compound of formula **II** to the acid, HX, is from 1:1 to 1:8. By reducing the amount of acid used the rate of reaction of step i) is decreased.
In addition, step i) according to the first aspect of the present invention is conducted in the presence of an organic solvent. Suitable organic solvents may be selected from alcohols, ethers, esters, chlorinated hydrocarbons and mixtures thereof and aqueous mixtures thereof, preferably the organic solvent is selected from the group consisting of alcohols, ethers and esters. Suitable alcohols include linear, cycled or branched C₁-C₆ alcohols, preferably methanol, ethanol, isopropanol and mixtures thereof. Suitable ethers include diethyl ether, tert-butyldimethyl ether, tetrahydrofuran, dioxane and mixtures thereof and aqueous mixtures thereof. Suitable esters include ethyl acetate, isopropyl acetate and mixtures thereof. Suitable chlorinated hydrocarbons include dichloromethane, chloroform and mixtures thereof. More preferably, the solvents used in step i) are methanol, ethanol, isopropanol and mixtures thereof, more preferably, methanol, ethanol and mixtures thereof. Also, step i) may be carried out by stirring the reaction mixture containing intermediate **II,** the acid and the suitable organic solvent for a period of time sufficient to form the compound of formula **III.**

The one-pot process of step i), i.e. removal of the R₂ protecting group and salt formation, is carried out at the refluxing temperature of the selected solvent or mixture of solvents that can vary from 25 °C to 150 °C depending on the solvent or solvents used. Preferably, the reaction temperature of step i) is from 50 °C to 100 °C. More preferably, the reaction temperature is from 60 °C to 80 °C which helps controlling the reaction rate of step i). Completion of the reaction can be monitored by methods known in the art, as TLC of HPLC. Then, after the reaction is substantially completed, the reaction mixture is further cooled down to a temperature between room temperature to 0 °C. The term room temperature in the context of the present invention means that the temperature is between 10 °C and 40 °C, preferably between 15 °C and 30 °C, more preferably between 20 °C and 25 °C.

In one particular embodiment in step i) of the first aspect the compound of formula **II** wherein R₁ is benzyl and R₂ is tert-butyldimethylsilyl is reacted with hydrochloric acid in the presence of an organic solvent to provide the corresponding hydrochloride salt.

In addition, the compound of formula **III**, preferably wherein R₁ is benzyl and HX is hydrochloric acid, may be further purified by known methods to those skilled in the art. Examples of conventional purification techniques which can be carried out on an industrial scale are solvent extraction, filtration, slurring, washing, phase separation, evaporation, centrifugation or crystallization.

Preferably, the compound of formula **III**, preferably wherein R₁ is benzyl and HX is hydrochloric acid, is purified by slurry. The purification is conducted by removing the solvent used in step i) and adding a second solvent which acts as antisolvent to provide a suspension. Suitable second solvents used are those in which compound of formula **III**, preferably wherein R₁ is benzyl and HX is hydrochloric acid, are insoluble. These solvents may be selected from alcohols, ketones, ethers and esters. Suitable alcohols include but are not limited to isopropanol, 2-metyl-1-propanol, 1-butanol, 2-butanol, 1-pentanol, 3-methyl-1-butanol, tert-butanol and mixtures thereof. Suitable ketones include acetone, methyl ethyl ketone and methyl isobutyl ketone. Suitable ethers include diethyl ether, isopropyl ether and *tert*-butyl methyl ether. Suitable esters include methyl acetate, ethyl acetate, isopropyl acetate, butyl acetate and isobutyl acetate. Preferably, the second solvent is selected from acetone or isopropanol and mixtures thereof. The slurry may be carried out at a temperature range from 0 °C to 80 °C. Afterwards, the suspension obtained may be further cooled down, when needed, from room temperature to about 0 °C.

The compound of formula **III**, preferably wherein R₁ is benzyl and HX is hydrochloric acid, may be isolated by means of known isolation techniques. The compound of formula **III**, preferably wherein R₁ is benzyl and HX is hydrochloric acid, may be purified and isolated as a solid, preferably, as a crystalline solid, which is advantageous since its manipulation and purification is easier to carry out, especially if an enrichment of the optical purity is desired.

In another more particular embodiment, the obtained hydrochloride salt of compound of formula **III**, wherein HX is hydrochloric acid and R₁ is benzyl, is isolated as a crystalline solid.

Optionally, compound of formula **III** can be converted to a compound of formula **III.B** in step ii) by treatment with a base in the presence of a solvent, wherein the solvent is a mixture of organic solvent a water. Suitable base of step ii) may be hydroxides of alkali or alkali earth metals, and carbonates and/or bicarbonates of alkali or alkali earth metals. Preferably, the base is selected from the group consisting of hydroxides, carbonates and bicarbonates of sodium, potassium, lithium, calcium, magnesium or caesium. The molar ratio of base to compound of formula **III** used in step ii) may be of at least 1:2.

In addition, in the solvent of step ii) wherein said solvent is a mixture of organic solvent and water, preferably the organic solvent is selected from the group consisting of alcohols, ketones, ethers, esters and aromatic hydrocarbons. Preferably, the organic solvents are esters and aromatic hydrocarbons. Suitable esters are methyl acetate, ethyl acetate, isopropyl acetate and butyl acetate. Suitable aromatic hydrocarbons are toluene and xylenes. The quantity of organic solvent and water used in step ii) may be at least the sufficient quantity to dissolve the compound of formula **III** and the base, respectively. Moreover, the ratio of organic solvent and water may be about 100:1. Preferably, the ratio of organic solvent and water may be about 50:1. More preferably, the ratio of organic solvent and water may be about 10:1. Even more preferably, the ratio of organic solvent and water may be about 1:1. In addition, the ratio of organic solvent and water may be about 1:10.

In a particular embodiment, compound of formula **III** is converted to a compound of formula **III.B** in step ii) by treatment with a base in the presence of a solvent, wherein the solvent is a mixture of organic solvent a water. Preferably, R₁ of compound **III** is benzyl and HX is hydrochloric acid.

The compound of formula **III.B** of step ii) may be further purified to increase the enantiomeric purity. Optionally, step ii) further comprises the treatment of the compound of formula **III.B** with a chiral organic acid HA selected from the group consisting of tartaric acid and derivates thereof in the presence of an organic solvent to provide a compound of formula **III.HA.**

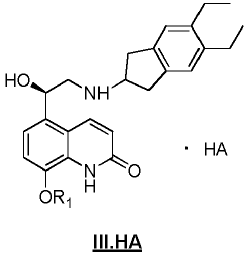

Suitable chiral organic acid HA is selected from the group consisting of L-tartaric acid, dibenzoyl-L-tartaric acid, dibenzoyl-D-tartaric acid, ditoluyl-L-tartaric acid and ditoluyl-D-tartaric acid. Suitable organic solvents are selected from the group consisting of alcohols, ketones, ethers and esters and mixtures thereof. Preferably, the organic solvent is selected from the group consisting of methanol, ethanol, isopropanol, acetone, methyl ethyl ketone, methyl isobutyl ketone, 3-pentanone and mixtures thereof. The molar ratio of chiral organic acid HA to compound of formula **III.B** is at least 1:2. Depending on the chiral organic acid used compounds of formula **III.HA** may be of hemi- (i.e. the molar ratio of chiral organic acid HA to compound of formula **III.B** is 1:2, i.e. 0.5:1), mono- (i.e. the molar ratio of chiral organic acid HA to compound of formula **III.B** is 1:1) or di-salts (i.e. the molar ratio of chiral organic acid HA to compound of formula **III.B** is 2:1). For example, when L-tartaric acid is used, the hemi-L-tartrate salt of compound **III.B** is preferably obtained, when ditoluyl-L-tartaric acid, ditoluyl-D-tartaric acid or hydrochloric acid are used, the corresponding monosalt of compound **III.B** is preferably obtained.

The treatment of compound **III.B** with a chiral organic acid to provide compound of formula **III.HA** may be carried out at the refluxing temperature of the selected solvent or mixture of solvents that can vary from 25 °C to 150 °C depending on the solvent or solvents used. Preferably, the reaction temperature of step i) is from 50 °C to 100 °C. More preferably, the reaction temperature is from 60 °C to 80 °C which helps controlling the reaction rate. Then, after the reaction is substantially completed, the reaction mixture is further cooled down to a temperature between room temperature to 0 °C. The term room temperature in the context of the present invention means that the temperature is between 10 °C and 40 °C, preferably between 15 °C and 30 °C, more preferably between 20 °C and 25 °C.

The compound of formula **III.HA** may be further converted into compound **III.B** by treatment with a base in the presence of a solvent, wherein the solvent is a mixture of organic solvent and water.

Advantageously, the use of tartaric acid and derivates thereof provide high chemical and enantiomeric purity and high yield of compounds of formula **III.HA** and **III.B.** Preferably, compound of formula **III.HA** wherein HA is L-tartaric acid provides significant improvement of the enantiomeric purity. On the other, hand, other organic acids used, as depicted in a comparative table in the experimental section, do not provide a significant enhancement of the enantiomeric purity nor the yield.

In a preferred embodiment, step ii) further comprises the treatment of compound of formula **III.B,** wherein R₁ is benzyl, with a chiral organic acid HA selected from the group consisting of L-tartaric acid, dibenzoyl-L-tartaric acid, dibenzoyl-D-tartaric acid, ditoluyl-L-tartaric acid and ditoluyl-D-tartaric acid, in the presence of an alcohol selected from the group of methanol, ethanol and isopropanol and/or ketones selected from the group of acetone, methyl ethyl ketone, methyl isobutyl ketone and 3-pentanone and/or mixtures thereof.

In a more preferred embodiment the compound of formula **III** of step i) is converted to a compound of compound **III.B** in step ii) by treatment with a base in the presence of a solvent, wherein the solvent is a mixture of organic solvent and water. In addition, the compound of formula **III.B,** wherein R₁ is benzyl, is treated with a chiral organic acid HA selected from the group consisting of L-tartaric acid, dibenzoyl-L-tartaric acid, dibenzoyl-D-tartaric acid, ditoluyl-L-tartaric acid and ditoluyl-D-tartaric acid to provide a compound of formula **III.HA,** in the presence of an alcohol selected from the group of methanol, ethanol and isopropanol and/or ketones selected from the group of acetone, methyl ethyl ketone, methyl isobutyl ketone and 3-pentanone and/or mixtures thereof. Preferably, the compound of formula **III.HA** may be further converted into compound **III.B** by treatment with a base in the presence of a solvent, wherein the solvent is a mixture of organic solvent and water. Preferably, R₁ of compound **III** is benzyl and HX is hydrochloric acid.

The deprotection step iii), wherein R₁ is removed from the products resulting from steps i) and ii), being the products the compounds of formula **III, III.B** and/or **III.HA,** preferably wherein HX is hydrochloric acid and R₁ is benzyl and preferably wherein HA is L-tartaric acid, the deprotection is conducted by means of hydrogenation. The hydrogenation is conducted in the presence of a catalyst and an organic solvent. Suitable catalysts are selected from Pd, Pt, Rh, Ru, Ni, Fe, Zn and Ir catalyst, such as, palladium (0) (Pd(0), palladium hydroxide (Pd(OH)₂), palladium on activated carbon (Pd/C), palladium on alumina, palladium on carbon powder, platinum, platinum on activated carbon and Raney™ nickel. A combination of catalysts may also be used. Most preferably, the catalyst is palladium on activated carbon. The amount of catalyst is not critical and may be equal or less than 10% by weight to the amount of compounds of formula **III, III.B** or **III.HA.** Moreover, the hydrogenation takes place at a hydrogen pressure range of from 0.5 to 10 atm. Most preferably, the hydrogen pressure is from about 0.5 to about 2 atm. The organic solvents used in step iii) may be polar and non-polar solvents. Preferably the solvent used in the deprotection step iii) is a polar solvent. Suitable polar solvents used in step iii) may be alcohols, esters, ethers, C₁-C₆ linear, branched or cyclic carboxylic acids, such as acetic acid, and mixtures thereof. Examples of alcohols include C₁-C₆ linear, branched or cyclic alcohols, such as methanol, ethanol, isopropanol, or mixtures thereof. Examples of esters include ethyl acetate, isopropyl acetate and mixtures thereof. Examples of ethers include tetrahydrofuran, dioxane and mixtures thereof. Preferably, the organic solvent is an alcohol selected from the group consisting of methanol, ethanol, isopropanol and mixtures thereof. More referably, the solvent is methanol, ethanol, mixtures thereof and mixtures of methanol and/or ethanol with water.

The deprotection step iii) may take place further in the presence of a base. The deprotection is preferably carried out in the presence of a base when R₁ is removed from the compounds of formula **III** and/or **III.HA.** Suitable bases used in step iii) are organic and inorganic bases. Suitable organic bases are carboxylates, such as formate, acetate and propionate salts of alkali or alkali earth metals and mixtures thereof. Suitable inorganic bases are carbonates and bicarbonates of alkali or alkali earth metals and mixtures thereof. Preferably , the base is selected from the group consisting of sodium formate, sodium acetate and sodium propionate. More referably, the suitable base used in step iii) is an organic base such as sodium acetate. The molar ratio of base to the compound of formula **III** or **III.HA** used in step iii) may be in a range of about 1 to 5. Preferably, the molar ratio of base used in step iii) to the compound of formula **III** or **III.HA** is in a range about 1 to 4. More preferably, the molar ratio of base used in step iii) to the compound of formula **III** or **III.HA** is from 2 to 3, since the yield of indacaterol formed is improved when using these conditions. The reaction temperature may take place over a range of temperature from 15 °C to refluxing temperature of selected solvent.

After completion of the reaction of step iii), which can be monitored by methods known in the art, such as TLC (Thin Layer Chromatography) of HPLC (High Performance Liquid Chromatography), the obtained indacaterol may be purified by means of conventional purification techniques.

In a particular embodiment, the compound of formula **III**, the compound of formula **III.B** and/or the compound of formula **III.HA** obtained in any of the steps i) or ii) are purified by means of conventional purification techniques. Examples of conventional purification techniques which can be carried out on an industrial scale are solvent extraction, filtration, slurring, washing, phase separation, evaporation, centrifugation or crystallization.

Preferably, the compound of formula **III.HB** wherein R₁ is benzyl and HX is hydrochloric acid is purified by conventional purification techniques which can be carried out on an industrial scale are solvent extraction, filtration, slurring, washing, phase separation, evaporation, centrifugation or crystallization.

Even more preferably, the compound of formula **III.HA,** wherein R₁ is benzyl and HA is L-tartaric acid is purified by conventional purification techniques which can be carried out on an industrial scale are solvent extraction, filtration, slurring, washing, phase separation, evaporation, centrifugation or crystallization.

In a particular embodiment, compound of formula **III.HA,** wherein HA is a chiral organic acid HA selected from the group consisting of L-tartaric acid, dibenzoyl-L-tartaric acid, dibenzoyl-D-tartaric acid, ditoluyl-L-tartaric acid and ditoluyl-D-tartaric acid and R₁ is benzyl, is isolated as a crystalline solid.

In a more particular embodiment, the compound of formula **III.HA,** wherein HA is L-tartaric acid and R₁ is benzyl, is isolated as a crystalline solid.

If desired, indacarerol can be converted into an acceptable salt or co-crystal thereof. Indacaterol can be reacted with a pharmaceutically acceptable acid suitable to provide a pharmaceutically acceptable salt or co-crystal thereof. Preferably, the indacaterol obtained is reacted in step iv) with a pharmaceutically acceptable acid in the presence of an organic solvent, water and mixtures thereof. Suitable pharmaceutically acceptable acids are selected from hydrochloric acid, hydrobromic acid, sulfuric acid, methansulfonic acid, ethansulfonic acid, esylate acid, p-toluensulfonic acid, formic acid, acetic acid, malonic acid, malic acid, succinic acid, fumaric acid, glycolic acid, maleic acid, citric acid, tartaric acid, 1-hydroxy-2-naphthoic acid and the like. Preferably, pharmaceutically acceptable acid is selected from hydrochloric acid, acetic acid, malonic acid, malic acid, succinic acid, fumaric acid, glycolic acid, maleic acid, citric acid, tartaric acid and 1-hydroxy-2-naphthoic acid. More preferably, the pharmaceutically acceptable acid is maleic acid, acetic acid and 1-hydroxy-2-naphthoic acid.
In one embodiment, the indacaterol obtained is reacted with maleic acid to provide indacaterol maleate.
In a preferred embodiment the maleate salt obtained is purified by recrystallization. In a particular preferred embodiment indacaterol maleate is recrystallized in ethanol.

The compounds of formula **II** are prepared comprising;
a) reacting compound of formula **X;** 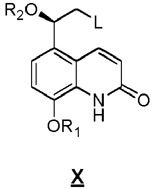 wherein R₁ and R₂ are as previously defined and L is a leaving group selected from halogens and sulfonates, with 2-amino-(5,6-diethyl)-indan to provide compound of formula **II,** wherein R₁ and R₂ are as previously defined.
   Or
a') reacting compound of formula **X**; 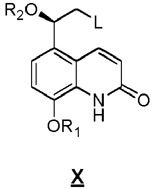 wherein R₁ and R₂ are as previously defined and L is a leaving group selected from halogens and sulfonates, with a salt of 2-amino-(5,6-diethyl)-indan in the presence of a base to provide the compound of formula **II,** wherein R₁ and R₂ are as previously defined.

The term leaving group means a functional group or an atom that can be displaced by another functional group or atom in a substitution reaction, such as a nucleophilic substitution reaction. The leaving group L of a compound of formula **X** is selected from halogens, alkyl, aryl, arylalkyl and aralkyl sulfonates. Suitable halogens are chloro, bromo and iodo. Suitable alkyl sulfonates may be mesylate. Suitable aralkyl sulfonates may be tosylate, brosylate (*p*-bromobenzensulfonate), nosylate (*p-*nitrobenzenesulfonate) and the like. Preferably, the leaving group is bromo and iodo.

The compound of formula **X** as a pure chiral compound with the R configuration can be obtained by known methods, or by analogy with known methods. For example, the compound of formula **X** wherein R₁ is Bn and R₂ is TBDMS is obtained by the synthetic method described in WO2003/042160. The compound 2-amino-(5,6-diethyl)-indan or its hydrochloride salt can be prepared as described in WO00/75114.

The process of step a) for preparing compounds of formula **II** may be conducted without solvent. Alternatively, the process of step a) may be carried out in the presence of a polar aprotic solvent. Suitable organic solvents are polar aprotic solvents, selected from the group of dimethylsufoxide, dimethylformamide, acetonitrile, N-methyl-pyrrolidinone, dioxane and the like.
The process of step a) may be carried out in the presence of a base. Suitable bases are those which act as an acid scavenger, which may be organic or inorganic bases. Suitable organic bases are tertiary and aromatic amines. Examples of tertiary amines include triethylamine, diisopropylethylamine and the like. Examples of aromatic amines include pyridine, imidazole and the like. Suitable inorganic bases are carbonates and bicarbonates of calcium, sodium, magnesium, potassium, lithium and caesium and mixtures thereof. Preferably, the base is an organic base, selected from pyridine and an inorganic base selected from carbonates and bicarbonates of sodium and potassium and mixtures thereof.
The process of step a) may be carried out in the presence of a catalyst. Suitable catalysts are those which facilitate the reaction of step a) by increasing the reaction rate. Preferably, the catalyst used is sodium iodide or potassium iodide.
The amount of polar aprotic solvent to compound of formula **II** used in step a) is in the range of 0.01 to 1 (v/w).

Preferably, the amount of the polar aprotic solvents to compound of formula **II** is in the range of 0.01 to 0.8 (v/w). More preferably, is in the range of 0.01 to 0.6 (v/w). By reducing the amount of the polar aprotic solvents used the reaction rate is increased.

The molar ratio of base to compound of formula **II** used in step a) is in a range of 1:1 to 5:1. Preferably, the molar ratio is in a range of 2:1 to 4:1.

The amount of catalyst to intermediate of formula **II** used in step a) is between 0.1 to 5% by weight. Preferably, the amount of catalyst to intermediate of formula **II** used is between 0.1 to 2% by weight.

The process of step a) is carried out by heating or subjecting the reaction to microwave radiation (e.g. 300 watts). This reaction may be conducted at a temperature in the range of 20 °C to 140 °C. Preferably, the process of step a) is carried out from 80 °C to 130 °C, more preferably from 100 °C to 130 °C. Upon the reaction is substantially completed, the reaction mixture is cooled down to a temperature between 70 °C to 50 °C.

The process of step a') for preparing compounds of formula **II** is carried out in the presence of an organic or an inorganic base. Suitable organic bases are tertiary and aromatic amines. Examples of tertiary amines include triethylamine, diisopropylethylamine and the like. Examples of aromatic amines include pyridine, imidazole and the like. Suitable inorganic bases are carbonates and bicarbonates of calcium, sodium, magnesium, potassium, lithium and caesium and mixtures thereof. Suitable bases are those which act as an acid scavenger, which may be organic or inorganic bases. Preferably, the base used in step a') is an organic base, selected from pyridine and an inorganic base selected from carbonates and bicarbonates of sodium and potassium and mixtures thereof.

The molar ratio of the base to compound of formula **II** used in step a') is of at least 1:1.

The process of step a') for preparing compounds of formula **II** may be conducted without solvent. Alternatively, the process of step a') may be carried out in the presence of a polar aprotic solvent. Suitable organic solvents are polar aprotic solvents, selected from the group of dimethylsufoxide, dimethylformamide, acetonitrile, N-methyl-pyrrolidinone, dioxane and the like. The amount of polar aprotic solvent to compound of formula **II** used in step a') is in the range of 1:0.01 to 1:1 (v/w). Preferably, the amount of the polar aprotic solvents to compound of formula **II** is in the range of 1:0.01 to 1:0.8 (v/w). More preferably, is in the range of 1:0.01 to 1:0.6 (v/w). By reducing the amount of the polar aprotic solvents used the reaction rate is increased.
The process of step a') may be carried out in the presence of a catalyst. Suitable catalysts are those which facilitate the reaction of step a) by increasing the reaction rate. Preferably, the catalyst used is sodium iodide or potassium iodide. The amount of catalyst to intermediate of formula **II** used in step a') is between 0.1 to 5% by weight. Preferably, the amount of catalyst with respect to intermediate of formula **II** used is between 0.1 to 2% by weight.

The process of step a') is carried out by heating or subjecting the reaction to microwave radiation (e.g. 300 watts). This reaction may be conducted at a temperature in the range of 20 °C to 140 °C. Preferably, the process of step a) is carried out from 80 °C to 130 °C, more preferably from 110 °C to 130 °C. Upon the reaction is substantially completed, the reaction mixture is cooled down to a temperature between 70 °C to 50 °C.
After completion of the reaction of step a) or a') the compounds of formula **II** may be isolated by conventional isolating techniques known in the art. Preferably, the compounds of formula **II** can be separated by extraction in organic solvents from an aqueous layer. Suitable organic solvents are selected from isopropyl acetate, tert-butyl methyl ether and dichloromethane. More preferably, compounds of formula **II** are extracted using isopropyl acetate. Thereafter, the solvent is removed and the compounds of formula **II** are preferably used directly in the next step without further purification. If desired, compounds of formula **II** may be purified by conventional purification techniques to those skilled in the art.

Also described herein is a solid crystalline form, Form I, of compound of formula **III**, wherein HX is hydrochloric acid and R₁ is benzyl, which is characterized by at least one of the following:
1. A powder X-ray diffraction (PXRD) pattern having characteristics peaks at approximately 4.3, 10.1, 11.9 and 23.6 ± 0.2° degrees two theta (i.e. Bragg's angle); or
2. A DSC thermogram showing an endothermic peak with an onset at approximately 245-248 °C, and a maximum at approximately 249-252 °C.
The term approximately means in the context of X-ray diffraction measurements that there is an uncertainty in the measurements of the degrees 2-theta of ± 0.2 (expressed in degrees 2-theta). The term approximately means in this context of DSC measurements that the °C values can vary by 2 °C, preferably by 1 °C. In the case of a temperature range preceded by the term approximately, it means that each endpoint of the range may vary by 2 °C, preferably by 1 °C.
In a particular embodiment, Form I of compound of formula **III**, wherein HX is hydrochloric acid and R₁ is benzyl, is further characterized in that the X-ray powder diffraction pattern further comprises the following peaks at approximately: 4.3, 8.6, 10.1, 11.9, 14.3, 17.3 and 23.6 ± 0.2° degrees two theta.

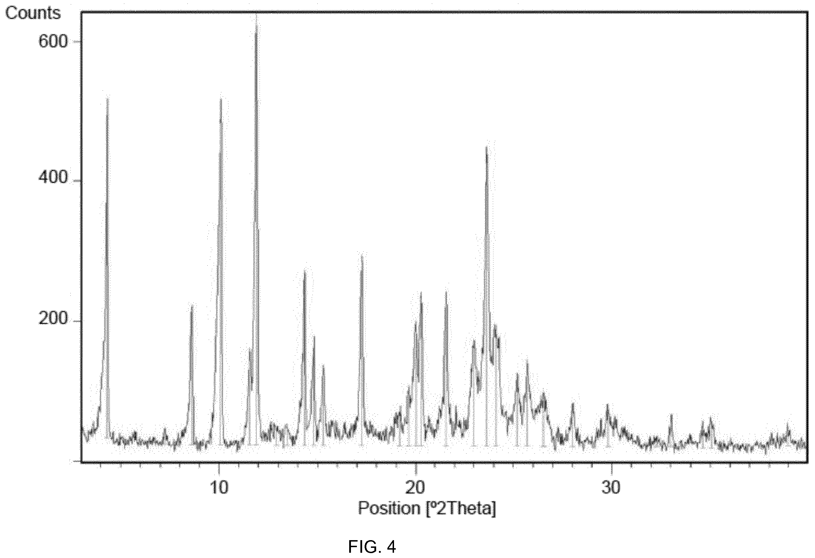

In a further embodiment, the Form I of compound of formula **III**, wherein HX is hydrochloric acid and R₁ is benzyl, is characterized in that it provides a
PXRD pattern substantially in accordance to Figure 4.

In a further embodiment, the polymorph of crystalline compound of formula **III**, wherein HX is hydrochloric acid and R₁ is benzyl, Form I, is characterized in that it provides a powder X-ray diffraction pattern, characterized by the interplanar distance values shown below:

| **Angle 2θ (°)** **(±0.2)** | **d value (Å)** |
|---|---|
| 4.3 | 20.5 |
| 8.6 | 10.2 |
| 10.1 | 8.8 |
| 11.6 | 7.6 |
| 11.9 | 7.4 |
| 14.3 | 6.2 |
| 14.8 | 5.9 |
| 15.3 | 5.8 |
| 17.3 | 5.1 |
| 19.6 | 4.5 |
| 20.0 | 4.4 |
| 20.2 | 4.3 |
| 21.5 | 4.1 |
| 22.9 | 3.9 |
| 23.6 | 3.8 |
| 24.0 | 3.7 |
| 25.2 | 3.5 |
| 25.7 | 3.4 |
| 26.5 | 3.3 |

In a further embodiment, the polymorph of crystalline compound of formula **III**, wherein HX is hydrochloric acid and R₁ is benzyl, Forml, is characterized in
that it provides a powder X-ray diffraction pattern, characterized by the interplanar distance values and relative intensity (in percentage) at approximately the values shown below:

| **Angle 2θ (°)** **(±0.2)** | **d value (Å)** | **Rel. Int. [%]** |
|---|---|---|
| 4.3 | 20.5 | 73.4 |
| 8.6 | 10.2 | 30.9 |
| 10.1 | 8.8 | 73.5 |
| 11.6 | 7.6 | 22.2 |
| 11.9 | 7.4 | 100.0 |
| 14.3 | 6.2 | 40.1 |
| 14.8 | 5.9 | 20.4 |
| 15.3 | 5.8 | 18.1 |
| 17.3 | 5.1 | 43.9 |
| 19.6 | 4.5 | 13.6 |
| 20.0 | 4.4 | 28.2 |
| 20.2 | 4.3 | 34.1 |
| 21.5 | 4.1 | 35.0 |
| 22.9 | 3.9 | 24.3 |
| 23.6 | 3.8 | 64.8 |
| 24.0 | 3.7 | 26.5 |
| 25.2 | 3.5 | 16.6 |
| 25.7 | 3.4 | 19.7 |
| 26.5 | 3.3 | 10.7 |

The term approximately means in this context of PXRD intensity measurements that there is an uncertainty in the measurements of the relative intensities. It is known to the person skilled in the art that the uncertainty of the relative intensities depends strongly on the measurement conditions. The relative intensity values can e.g. vary by 30%.

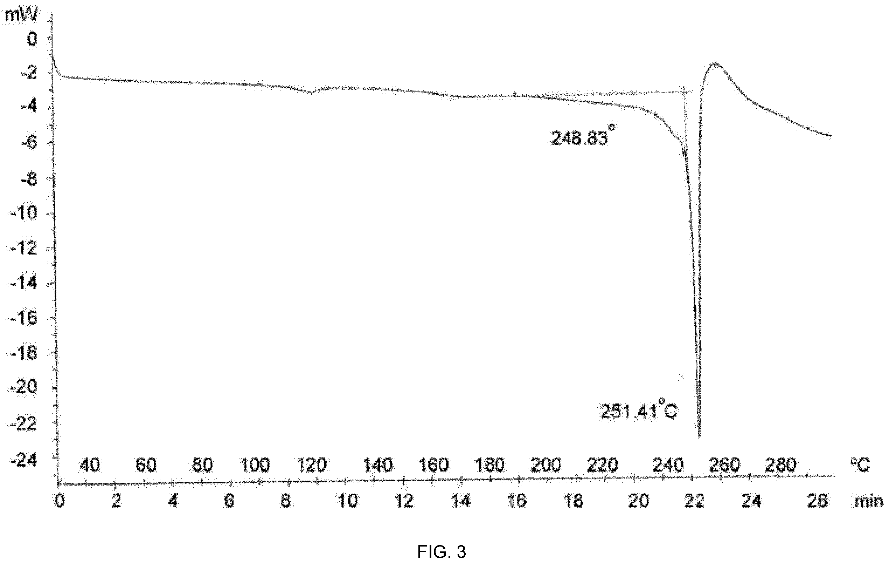

In a further embodiment, the polymorph of a crystalline compound of formula **III**, wherein HX is hydrochloric acid and R₁ is benzyl, polymorph I, is characterized in that it provides a DSC substantially in accordance to Figure 3.

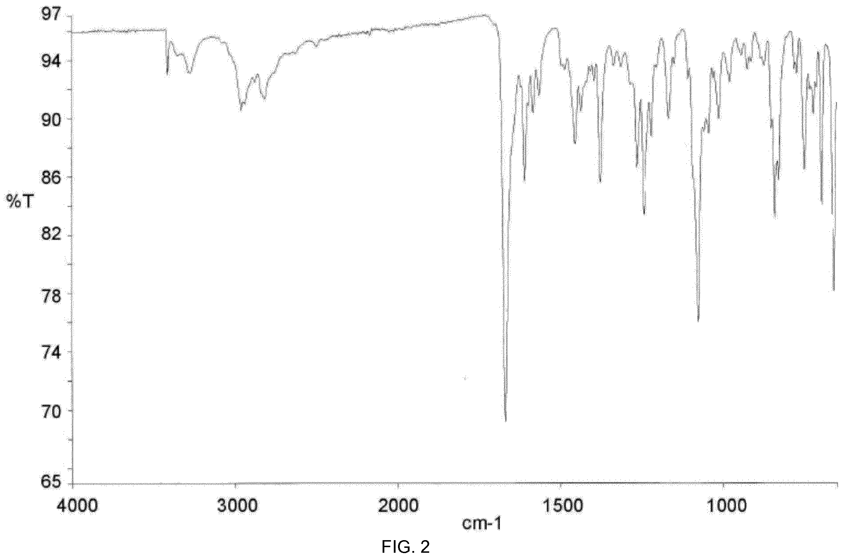

In a further embodiment, the polymorph I of a crystalline compound of formula **III**, wherein HX is hydrochloric acid and R₁ is benzyl, is characterized in that it provides an IR substantially in accordance to Figure 2.

Also described herein is a solid crystalline form of compound of formula **III.HA,** in a molar ratio of HA to III of 1:2, wherein HA is L-tartaric acid and R₁ is benzyl, characterized by at least one of the following:
1. An IR having the characteristics bands at approximately 3376, 3313, 3054, 2964, 1659, 1597, 1339, 1059, 833 and 740 cm⁻¹;
   or
2. A DSC thermogram showing a small endothermic peak with a maximum at approximately 160 °C, and a sharp endotherm with a maximum at approximately 210 °C.
The term approximately means in this context of IR measurements that the cm⁻¹ values can vary by 4 cm⁻¹, preferably by 2 cm⁻¹. The term approximately means in this context of DSC measurements that the °C values can vary by 2 °C, preferably by 1 °C. In the case of a range preceded by the term approximately, it means that each endpoint of the range may vary by 2 °C, preferably by 1 °C.

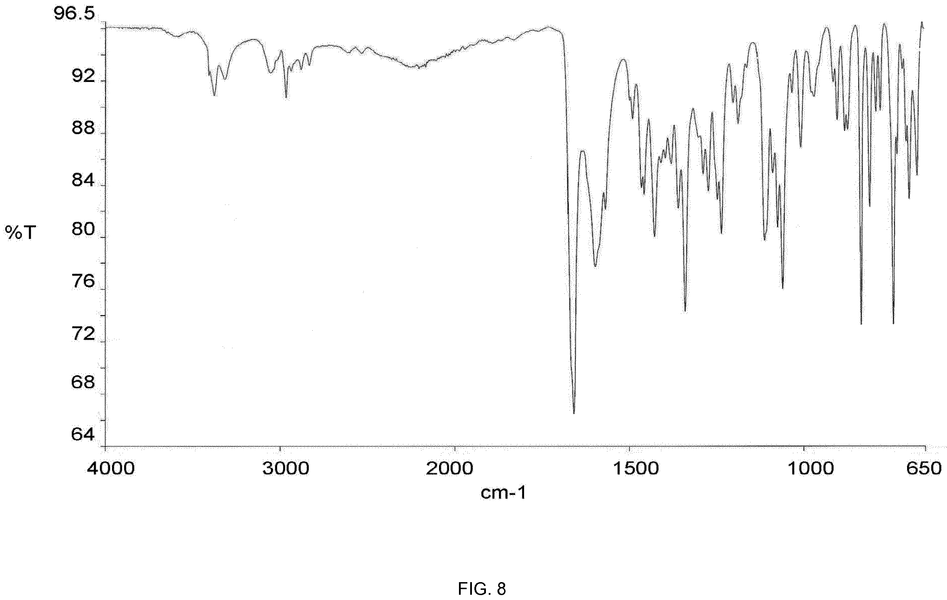

In a further embodiment, the solid crystalline form of compound of formula **III.HA,** in a molar ratio of HA to III of 1:2, wherein HA is L-tartaric acid and R₁ is benzyl, is characterized in that it provides an IR analysis substantially in accordance to Figure 8.

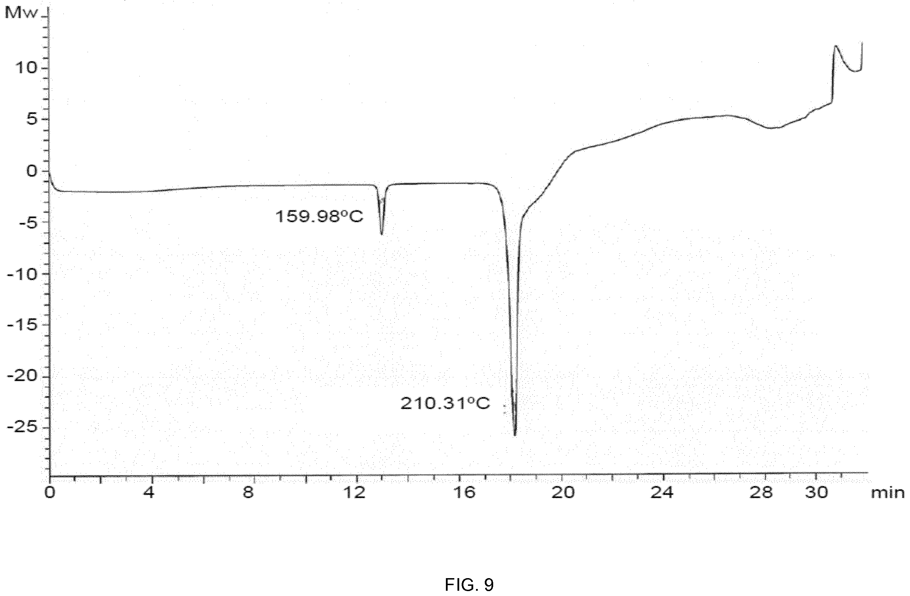

In a further embodiment, the solid crystalline form of compound of formula **III.HA,** in a molar ratio of HA to III of 1:2, wherein HA is L-tartaric acid and R₁ is benzyl, is characterized in that it provides a DSC substantially in accordance to Figure 9.

Also described herein is a solid crystalline form of compound of formula **III.HA,** wherein HA is ditoluyl-L-tartaric acid and R₁ is benzyl, characterized by at least one of the following:
1. An IR having the characteristics bands at approximately 3404, 2964, 2932, 1717, 1668, 1609, 1266, 1083, 1107 and 751 cm⁻¹;
   or
2. A DSC thermogram showing an endothermic peak with a maximum at approximately 186 °C.
The term approximately means in this context of IR measurements that the cm⁻¹ values can vary by 4 cm⁻¹, preferably by 2 cm⁻¹. The term approximately means in this context of DSC measurements that the °C values can vary by 2 °C, preferably by 1 °C. In the case of a temperature range preceded by the term approximately, it means that each endpoint of the range may vary by 2 °C, preferably by 1 °C.

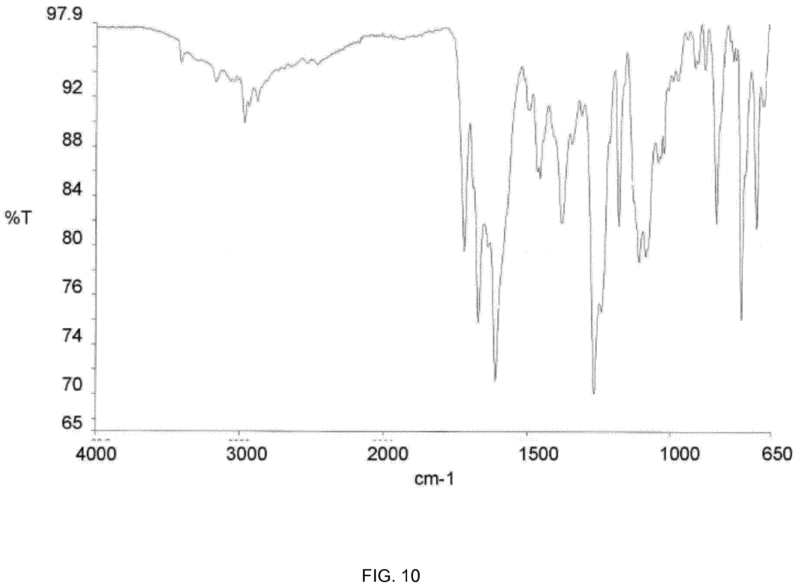

In a further embodiment, the solid crystalline form of compound of formula **III.HA,** wherein HA is ditoluyl-L-tartaric acid and R₁ is benzyl, is characterized in
that it provides an IR analysis substantially in accordance to Figure 10.

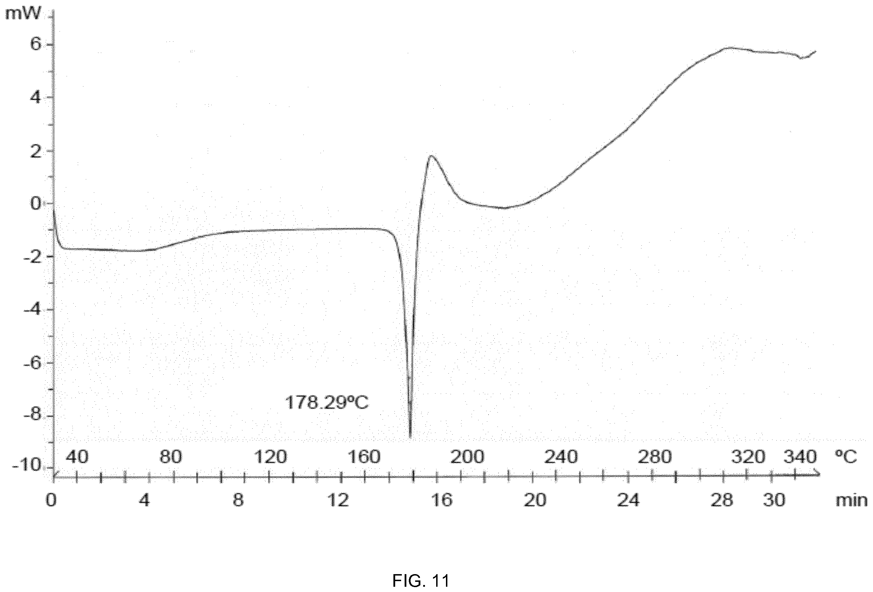

In a further embodiment, the solid crystalline form of compound of formula **III.HA,** wherein HA is ditoluyl-L-tartaric acid and R₁ is benzyl, is characterized in
that it provides a DSC substantially in accordance to Figure 11.

Also described herein is a solid crystalline form of compound of formula **III.HA,** wherein HA is ditoluyl-D-tartaric acid and R₁ is benzyl, characterized by at least one of the following:
1. An IR having the characteristics bands at approximately 2966, 2928, 1726, 1703, 1655, 1608, 1266, 1110 and 747 cm⁻¹;
   or
2. A DSC thermogram showing an endothermic peak with a maximum at approximately 178 °C.

The term approximately means in this context of IR measurements that the cm⁻¹ values can vary by 4 cm⁻¹, preferably by 2 cm⁻¹. The term approximately means in this context of DSC measurements that the °C values can vary by 2 °C, preferably by 1 °C. In the case of a temperature range preceded by the term approximately, it means that each endpoint of the range may vary by 2 °C, preferably by 1 °C.

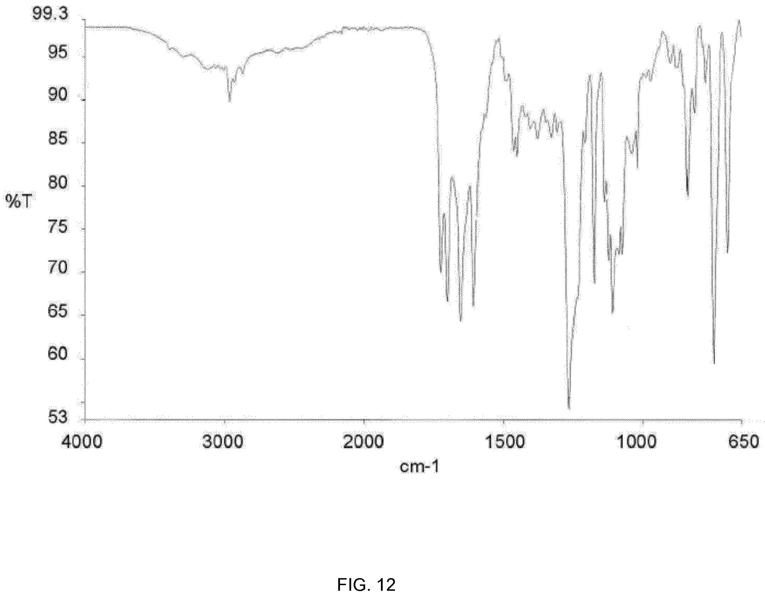

In a further embodiment, the solid crystalline form of compound of formula **III.HA,** wherein HA is ditoluyl-D-tartaric acid_and R₁ is benzyl, is characterized in
that it provides an IR analysis substantially in accordance to Figure 12.

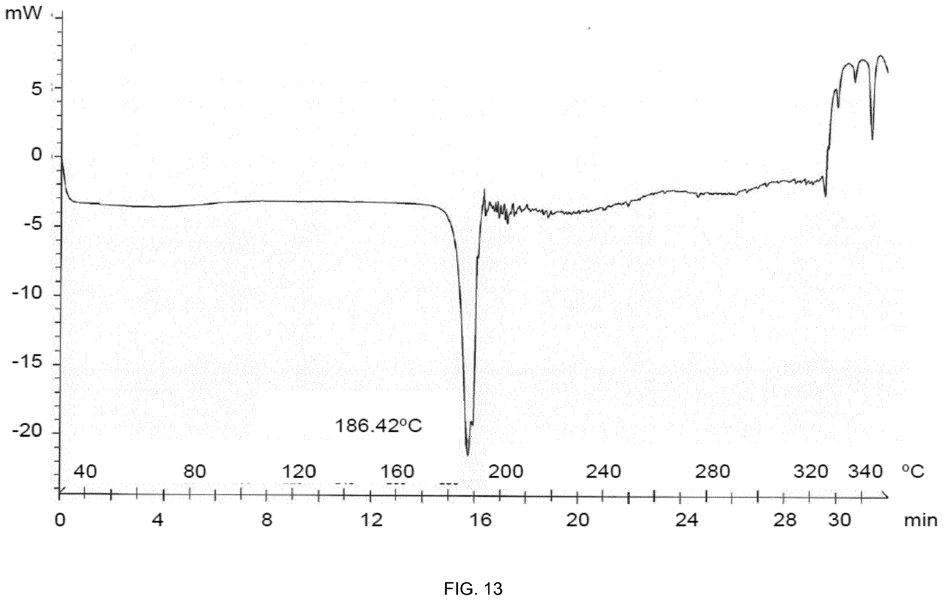

In a further embodiment, the solid crystalline form of compound of formula **III.HA,** wherein HA is ditoluyl-D-tartaric acid and R₁ is benzyl, is characterized in
that it provides a DSC substantially in accordance to Figure 13.

In the following, the present invention is further illustrated by examples. They should in no case be interpreted as a limitation of the scope of the invention as defined in the claims.

### DESCRIPTION OF PREFERRED EMBODIMENTS

The present invention in particular is directed to the following embodiments.

A process for manufacturing indacaterol or a pharmaceutically acceptable salt or co-crystal thereof, which comprises at least the steps of:
i) reacting compound of formula **II**, wherein R₁ is an aralkyl group and R₂ is a silyl group, with an acid, HX, preferably wherein HX is hydrochloric acid, in the presence of an organic solvent to provide a compound of formula **III**, wherein R₁ is as defined above,
ii) converting compound of formula **III** to a compound of formula **III.B** by treatment with a base in the presence of a solvent, wherein the solvent is a mixture of an organic solvent and water,
iii) removing the protecting group R₁ from the products resulting from step i) or ii), when present, by hydrogenation, in the presence of a catalyst and an organic solvent to provide indacaterol of formula **I,** wherein R₁ is as defined above,
iv) optionally, treating the indacaterol of formula **I** obtained in step iii) with a pharmaceutically acceptable acid to provide a pharmaceutically acceptable salt or co-crystal of indacaterol.

A process for manufacturing indacaterol or a pharmaceutically acceptable salt or co-crystal thereof, which comprises at least the steps of:
i) reacting compound of formula **II**, wherein R₁ is benzyl or *p*-methoxybenzyl and R₂ is triethylsilyl or tert-butyldimethylsilyl with an
   acid, HX, preferably wherein HX is hydrochloric acid, in the presence of an organic solvent to provide a compound of formula **III**, wherein R₁ is as defined above,
ii) converting compound of formula **III** to a compound of formula **III.B** by treatment with a base in the presence of a solvent, wherein the solvent is a mixture of an organic solvent and water,
iii) removing the protecting group R₁ from the products resulting from step i) or ii), when present, by hydrogenation, in the presence of a catalyst and an organic solvent to provide indacaterol of formula **I,** wherein R₁ is as defined above,
iv) optionally, treating the indacaterol of formula **I** obtained in step iii) with a pharmaceutically acceptable acid to provide a pharmaceutically acceptable salt or co-crystal of indacaterol.

A process for manufacturing indacaterol or a pharmaceutically acceptable salt or co-crystal thereof, which comprises at least the steps of:
i) reacting compound of formula **II**, wherein R₁ is benzyl or *p*-methoxybenzyl and R₂ is triethylsilyl or tert-butyldimethylsilyl with an
   acid, HX, preferably wherein HX is hydrochloric acid, in the presence of an organic solvent to provide a compound of formula **III**, wherein R₁ is as defined above,
ii) converting compound of formula **III** to a compound of formula **III.B** by treatment with a base in the presence of a solvent, wherein the solvent is a mixture of an organic solvent and water, and treating said compound of formula **III.B** with a chiral organic acid HA selected from the group consisting of tartaric acid and derivates thereof in the presence of an organic solvent to provide a compound of formula **III.HA,**
iii) removing the protecting group R₁ from the products resulting from step i) or ii), when present, by hydrogenation, in the presence of a catalyst and an organic solvent to provide indacaterol of formula **I,** wherein R₁ is as defined above,
iv) optionally, treating the indacaterol of formula **I** obtained in step iii) with a pharmaceutically acceptable acid to provide a pharmaceutically acceptable salt or co-crystal of indacaterol.

A process for manufacturing indacaterol or a pharmaceutically acceptable salt or co-crystal thereof, which comprises at least the steps of:
i) reacting compound of formula **II**, wherein R₁ is benzyl or *p*-methoxybenzyl and R₂ is triethylsilyl or tert-butyldimethylsilyl with an
   acid, HX, preferably wherein HX is hydrochloric acid, in the presence of an organic solvent to provide a compound of formula **III**, wherein R₁ is as defined above,
ii) converting compound of formula **III** to a compound of formula **III.B** by treatment with a base in the presence of a solvent, wherein the solvent is a mixture of an organic solvent and water, and treating said compound of formula **III.B** with a chiral organic acid HA selected from the group consisting of L-tartaric acid, dibenzoyl-L-tartaric acid, dibenzoyl-D-tartaric, ditoluyl-L-tartaric acid and ditoluyl-D-tartaric in the presence of an organic solvent to provide a compound of formula **III.HA,**
iii) removing the protecting group R₁ from the products resulting from step i) or ii), when present, by hydrogenation, in the presence of a catalyst and an organic solvent to provide indacaterol of formula **I,** wherein R₁ is as defined above,
iv) optionally, treating the indacaterol of formula **I** obtained in step iii) with a pharmaceutically acceptable acid to provide a pharmaceutically acceptable salt or co-crystal of indacaterol.

A process for manufacturing indacaterol or a pharmaceutically acceptable salt or co-crystal thereof, which comprises at least the steps of:
i) reacting compound of formula **II**, wherein R₁ is benzyl or *p*-methoxybenzyl and R₂ is triethylsilyl or tert-butyldimethylsilyl with an
   acid, HX, preferably wherein HX is hydrochloric acid, in the presence of an organic solvent to provide a compound of formula **III**, wherein R₁ is as defined above,
ii) converting compound of formula **III** to a compound of formula **III.B** by treatment with a base in the presence of a solvent, wherein the solvent is a mixture of an organic solvent and water, and treating said compound of formula **III.B** with a chiral organic acid HA selected from the group consisting of L-tartaric acid, dibenzoyl-L-tartaric acid, dibenzoyl-D-tartaric, ditoluyl-L-tartaric acid and ditoluyl-D-tartaric in the presence of an organic solvent to provide a compound of formula **III.HA,** and converting the compound **III.HA** into compound of formula **III.B** by treatment with a base in the presence of a solvent, wherein the solvent is a mixture of an organic solvent and water,
iii) removing the protecting group R₁ from the products resulting from step i) or ii), when present, by hydrogenation, in the presence of a catalyst and an organic solvent to provide indacaterol of formula **I,** wherein R₁ is as defined above
iv) optionally, treating the indacaterol of formula **I** obtained in step iii) with a pharmaceutically acceptable acid to provide a pharmaceutically acceptable salt or co-crystal of indacaterol, preferably the pharmaceutically acceptable acid is maleic acid, acetic acid and 1-hydroxy-2-naphtoic acid.

In another particular embodiment of the first aspect of the present invention provides a process for manufacturing indacaterol or a pharmaceutically acceptable salt or co-crystal thereof, comprising at least the steps of:
i) reacting compound of formula **II,** wherein R₁ in benzyl and R₂ is tert-butyldimethylsilyl, with an acid, HX, wherein the acid is hydrochloric acid in the presence of an organic solvent to provide a compound of formula **III**, wherein R₁ and HX are as defined above and purifying the corresponding compound of formula **III** by slurring,
ii) optionally converting compound of formula **III** to a compound of formula **III.B** by treatment with a base in the presence of a solvent, wherein the solvent is a mixture of an organic solvent and water,
iii) removing the protecting group R₁ from the products resulting from step i) or ii) by hydrogenation in the presence of a catalyst, a base and an organic solvent to provide indacaterol, where in R₁ is as defined above,
iv) optionally, treating the indacaterol obtained in step iii) with a pharmaceutically acceptable acid to provide a pharmaceutically acceptable salt or cocrystal of indacaterol

In another particular embodiment of the first aspect of the present invention provides a process for manufacturing indacaterol or a pharmaceutically acceptable salt thereof, comprising at least the steps of:
i) reacting compound of formula **II,** wherein R₁ in benzyl and R₂ is tert-butyldimethylsilyl, with an acid, HX, wherein the acid is hydrochloric acid in the presence of an organic solvent to provide a compound of formula **III,** wherein R₁ and HX are as defined above and purifying the corresponding compound of formula **III** by slurring
ii) optionally converting compound of formula **III** to a compound of formula **III.B** by treatment with a base in the presence of a solvent, wherein the solvent is a mixture of an organic solvent and water,
iii) removing the protecting group R₁ from the products resulting from step i) or ii) by hydrogenation in the presence of a catalyst, a base and an organic solvent to provide indacaterol, where in R₁ is as defined above,
iv) optionally, treating the indacaterol obtained in step iii) with maleic acid to provide indacaterol maleate.

### EXPERIMENTAL

### General methods

### Proton Nuclear Magnetic Resonance (¹H NMR)

Proton nuclear magnetic resonance analyses were recorded in a deuterated solvent in a Varian Gemini 200 Fourier-Transform (FT) NMR spectrometer and chemical shifts are given in part per million (ppm) downfield from tetramethylsilane as internal standard. The coupling constants are given in Hz. Spectra were acquired dissolving 5-10 mg of sample in 0.7 mL of deuterated solvent.

### Infrared spectrometry (IR)

Infrared spectrometry analyses were recorded in a Perkin Elmer FT-IR spectrum One appliance using a Perkin Elmer ATR accessory.

### Differential Scanning Calorimetry (DSC)

DSC analyses were recorded in a Mettler Toledo DSC822e calorimeter. Experimental conditions: 40 µL aluminium crucibles; atmosphere of dry nitrogen at 50 mL/min flow rate; heating rate of 10 °C/min between 30 and 300°C. Data collection and evaluation was done with software STARe.

### Powder X-Ray Diffraction (PXRD)

Powder X-Ray Diffraction patterns were acquired using a Philips X'Pert powder diffractometer, equipped with a CuKα source (λ = 1.541874A; Kα2/a1 = 0.5), and a proportional detector, operating at 50 kV and 40 mA. Each sample was scanned between 3° and 40° in 28, with a step size of 0.03° and a scan rate of 1 s/step. Data collection carried out with High Score Plus.

### Examples

### Example 1. Preparation of (R)-8-(benzyloxy)-5-[1-((tert-butyidimethylsilyl)oxy)-2-((5,6-diethyl-2,3-dihydro-1H-inden-2-yl)amino)ethyl]quinolin-2(1H)-one

8-benzyloxy-5-(1*R*)-2-bromo-1-[*tert*-butyldimethylsilyloxy]-ethyl-quinolin-2-(1H)-one (12 g, 24.6 mmol), 2-amino-(5,6-diethyl)-indan hydrochloride (6.1 g, 27.0 mmol), sodium bicarbonate (5.2 g, 61.4 mmol) and of sodium iodide (0.37 g, 2.5 mmol) were charged into a reaction vessel. DMSO (6 ml) was added and the mixture obtained was stirred under inert atmosphere at 120 °C for 4 hours. Then, the resulting suspension was cooled down to 60 °C while stirring followed by addition of 50 mL of isopropyl acetate and 50 mL of water onto the reaction crude. The organic extract was washed with water and concentrated under reduce pressure to give (*R*)-8-(benzyloxy)-5-[1-((*tert-*butyldimethylsilyl)oxy)-2-((5,6-diethyl-2,3-dihydro-1 H-inden-2-yl)amino)ethyl]quinolin-2(1 H)-one as an oil, which was used in the next step without further purification.
Yield: 16 g
¹H NMR (200MHz, CDCl₃):
δ/ppm (TMS)= - 0.19 (s, 3H), 0.06 (s, 3H), 0.86 (s, 9H), 1.19 (t, *J* = 7 Hz, 6H), 2.61 (q, *J* = 7 Hz, 4H), 3.19 - 2.65 (m, 6H), 3.66 (m, 1 H), 5.17 (s, 2H), 5.20 (m, 1 H), 6.68 (d, *J* = 10 Hz), 7.00 (s, 2H), 7.01 (d, *J* = *8* Hz, 1H), 7.16 (d, *J* = 8 Hz, 1 H), 7.42 (m, 5H), 8.34 (d, *J* = 10 Hz, 1 H), 9.22 (s, 1 H).
IR (cm⁻¹): 3399, 2957, 2928, 2855, 1659, 1608, 1566, 1487, 1454, 1372, 1250, 1169, 1073, 1004, 957, 939, 908, 872, 832, 776, 732, 694, 666.

### Example 2. Preparation of (R)-8-(benzyloxy)--5-[2-(5,6-diethyl-indan-2-ylamino-1-hydroxyethyl]-1H-quinolin-2-one hydrochloride

(*R*)-8-(benzyloxy)-5-(1-[(tert-butyldimethylsilyl)oxy]-2-[(5,6-diethyl-2,3-dihydro-1H-inden-2-yl)amino)ethyl]quinolin-2(1 H)-one (5.3 g, 10 mmol) was dissolved in 26.5 mL of hydrochloric acid in ethanol (prepared from the mixture of 3.0 mL of HCl 37% and 23.5 mL of ethanol) at room temperature. The solution obtained was stirred at this temperature until the reaction was completed. Then, the formed suspension was cooled down to 0-5 °C and further stirred for 1 hour. The resulting solid was filtered off and washed with a cold mixture of ethanol-water (4:1). Afterwards, the solid obtained was dried under vacuum (2 mbar) at 50 °C for 4 hours.
Yield: 3.3 g, 79%
Purity (HPLC): 97.6%
¹H NMR (200MHz, DMSO-*d6*):
δ/ppm (TMS)= 1.10 (t, *J* = 8 Hz, 6H), 2.51 (q, *J* = 8 Hz, 4H), 3.17-3.06 (m, 6H), 3.98 (m, 1H), 5.31 (s, 2H), 5.59 (m, 1 H), 6.60 (d, *J =* 10 Hz, 1 H), 6.98 (s, 2H), 7.30-7.60 (m, 7H), 8.38 (d, *J =* 10 Hz), 9.18 (bs, 1 H), 10.25 (bs, 1 H), 10.83 (bs, 1 H).
IR (cm⁻¹): 3409, 3303, 2962, 2936, 2876, 2818, 1669, 1610, 1596, 1583, 1564, 1518, 1487, 1454, 1436, 1377, 1302, 1288, 1267, 1242, 1230, 1220, 1169, 1107, 1075, 1011, 976,839,749,695.
DSC: Weak first endotherm with a maximum at 137°C and second sharp endotherm with an onset at 248 °C and a maximum at 251 °C.
PXRD pattern substantially depicted as in Figure 4 (Form I)

### Example 3. Purification of (R)-8-(benzyloxy)-5-[2-(5,6-diethyl-indan-2-ylamino-1-hydroxyethyl]- 1H-quinolin-2-one hydrochloride

(*R*)-8-benzyloxy-5-[2-((5,6-diethyl-2,3-dihydro-1H-inden-2yl)-amino)-1-hydroxyethyl]-quinolin-2-(1 H)-one hydrochloride (5.00 g, 9.6 mmol, 97.6% purity) was suspended in isopropanol (25 mL) and stirred at 50 °C for 4 hours. The suspension was left to cool down to room temperature and further stirred for 12 hours. Then, the resulting solid was filtered off, washed with cold isopropanol and dried at 50 °C for 4 hours.
Yield: 4.7 g, 94%
Purity (HPLC): 99.2%
¹H NMR (200MHz, DMSO-*d6*):
δ/ppm (TMS)= 1.11 (t, *J* = 8 Hz, 6H), 2.54 (q, *J* = 8 Hz, 4H), 3.27 -2.99 (m, 6H), 4.00 (m, 1 H), 5.32 (s, 2H), 5.60 (m, 1 H), 6.60 (d, *J* = 10 Hz, 1 H), 6.99 (s, 2H), 7.30-7.60 (m, 7H), 8.41 (d, *J =* 10 Hz), 9.22 (s, 1H, NH).
IR (cm⁻¹): 3411, 3333, 2961, 2837, 1668, 1609, 1583, 1565, 1488, 1454, 1435, 1377, 1265, 1243, 1220, 1168, 1106, 1075, 976, 827, 779, 695.

### Example 4. Purification of (R)-8-(benzyloxy)-5-[2-(5,6-diethyl-indan-2-ylamino-1-hydroxyethyl]-1H-quinolin-2-one hydrochloride

(*R*)-8-benzyloxy-5-[2-((5,6-diethyl-2,3-dihydro-1H-inden-2yl)-amino)-1-hydroxyethyl]-quinolin-2-(1 H)-one hydrochloride (5.00 g, 9.6 mmol, 97.6% purity) was suspended in acetone (25 mL) and stirred at 50 °C for 4 hours. The suspension was left to cool down to room temperature and further stirred for 12 hours. Then, the resulting solid was filtered off, washed with cold acetone and dried at 50 °C for 4 hours.
Yield: 4.8 g, 95%
Purity (HPLC): 98.5%
¹H NMR (200MHz, DMSO-*d6*):
δ/ppm (TMS)= 1.11 (t, *J* = 8 Hz, 6H), 2.54 (q, *J* = 8 Hz, 4H), 3.27 -2.99 (m, 6H), 4.00 (m, 1 H), 5.32 (s, 2H), 5.60 (m, 1H), 6.60 (d, *J* = 10 Hz, 1 H), 6.99 (s, 2H), 7.30-7.60 (m, 7H), 8.41 (d, *J* = 10 Hz), 9.22 (s, 1H, NH).
IR (cm-¹): 3411, 3272, 2958, 2814, 1669, 1609, 1583, 1564, 1485, 1454, 1436, 1377, 1265, 1242, 1218, 1167, 1107, 1076, 977, 941, 923, 871, 839, 828, 749, 696, 659. DSC: Sharp endotherm with an onset at 249 °C and a maximum at 252 °C.
PXRD pattern substantially depicted as in Figure 4 (Form I)

### Example 5. Preparation of (R)-5-[2-(5,6-diethyl-indan-2-ylamino-1-hydroxyethyl]-8-hydroxy-1H-quinolin-2-one (2Z)-2-butenedioate

(*R*)-5-[2-(5,6-diethyl-indan-2-ylamino-1-hydroxyethyl]- 8-benzyloxy-1H-quinolin-2-one hydrochloride (5.00 g, 9.6 mmol) was mixed with 10% Pd/C (750 mg), sodium acetate (1.75 g, 21.3 mmol) and 96% ethanol (40 mL). The mixture was hydrogenated under 1 atm of H₂ at 50°C for 80 min. The Pd/C was filtered through a Celite pad. The filtrate was evaporated and the residue was dissolved in 96% ethanol (30 mL). The solution was heated to 60 °C. A solution of maleic acid (1.66 g, 14.3 mmol) in 96% ethanol (3.5 mL) was added. The mixture was cooled to 0-5 °C and stirred at this temperature for 1 hour. The suspension was filtered and washed with cold 96% ethanol. The white solid obtained was dried under vacuum (2 mbar) at 50 °C for 4 hours.
Yield: 3.9 g, 80%
Purity (HPLC): 97%
Enantiomeric purity (HPLC method): 99.1%
¹H NMR (200MHz, DMSO-*d6*):
δ/ppm(TMS)= 1.13 (t, *J* = 7 Hz, 6H), 2.57 (q, *J* = 7 Hz, 4H), 3.16 (m, 6H), 4.07 (m, 1 H), 6.02 (s, 2H), 6.61 (d, *J* = 9 Hz, 1 H), 6.99 (d, *J* = 9 Hz), 7.03 (s, 2H), 7.19 (d, *J* = 9 Hz), 8.17 (d, *J* = 9 Hz, 1 H).
FT-IR (cm⁻¹): 3391, 3338, 2964, 2873, 1877, 1663, 1637, 1602, 1533, 1472, 1438, 1384, 1361, 1246, 1226, 1190, 1150, 1124, 1083, 1032, 954, 869, 831, 783.
DSC: Sharp endotherm with an onset at 197 °C and a maximum at 204 °C.

Phase characterization by PXRD analysis of indacaterol maleate prepared matches that described in prior art WO08/25816.

### Example 6. Preparation of (R)-8-(benzyloxy)-5-[2-(5,6-diethyl-indan-2-ylamino-1-hydroxyethyl]-1H-quinolin-2-one hemi-L-tartrate

A solution of L-tartaric acid (345 mg, 2.3 mmol) in methanol (1,5 mL) was added to a solution comprising (*R*)-8-(benzyloxy)-5-[2-(5,6-diethyl-indan-2-ylamino-1-hydroxyethyl]-1H-quinolin-2-one (Compound III.B) (1,0 g, 2.0 mmol) in 9 mL of a mixture of methanol/acetone (2:1) at room temperature. The resulting mixture was stirred at the aforementioned temperature for 90 min. The resulting solid was filtered off and washed with a mixture of methanol/acetone (2:1). Afterwards, the solid obtained was dried at 40°C under atmosphere pressure for 15 hours.
Yield: 819 mg, 82%
Purity (HPLC): 99.4%
Enantiomeric purity (HPLC method): 99.5%
¹H NMR (200MHz, DMSO-*d6*):
δ/ppm (TMS)= 1.11 (t, *J* = 8 Hz, 6H), 2.54 (q, *J* = 8 Hz, 4H), 2.79-3.17 (m, 6H), 3.19 (t, *J* = 6 Hz, 1 H), 3.98 (s, 1 H), 5.31 (m, 4H), 6.59 (d, *J* = 10 Hz, 1 H), 6.96 (s, 2H), 7.22-7.57 (m, 9H), 8.25 (d, *J* = 10 Hz, 1 H).
IR (cm⁻¹): 3376, 3313, 3054, 2964, 2881, 2929, 1659, 1597, 1426, 1339, 1234, 1111, 1059,833,740.
DSC: Small endothermic peak with a maximum at approximately 160 °C, and a sharp endotherm with a maximum at approximately 210 °C.

### Example 7. Preparation of (R)-8-(benzyloxy)-5-[2-(5,6-diethyl-indan-2-ylamino-1-hydroxyethyl]-1H-quinolin-2-one ditoluyl-L-tartrate

A mixture of (*R*)-8-(benzyloxy)-5-[2-(5,6-diethyl-indan-2-ylamino-1-hydroxyethyl]-1H-quinolin-2-one (Compound III.B) (1.08 g, 2.08 mmol) and ditoluyl-L-tartaric (803 mg, 2.08 mmol) were dissolved in 50 mL of ethanol at reflux and stirred for 30 min. The resulting mixture was cooled down to room temperature and stirred for 3 hours. Then, the resulting solid was filtered off and washed with cold ethanol. Afterwards, the solid obtained was dried at 40°C under atmosphere pressure for 15 hours.
Yield: 1 g
Purity (HPLC): 99.9%
Enantiomeric purity (chiral HPLC method): 98.4%
IR (cm⁻¹): 3404, 3162, 2964, 2932, 2873, 1717, 1668, 1609, 1453, 1376, 1266, 1177, 1083, 1107, 837 and 751.
DSC: Sharp endotherm peak with a maximum at approximately 186 °C.

### Example 8. Preparation of (R)-8-(benzyloxy)-5-[2-(5,6-diethyl-indan-2-ylamino-1-hydroxyethyl]-1H-quinolin-2-one ditoluyl-D-tartrate

A mixture of (R)-8-(benzyloxy)-5-[2-(5,6-diethyl-indan-2-ylamino-1-hydroxyethyl]-1 H-quinolin-2-one (Compound III.B) (1.08 g, 2.08 mmol) and ditoluyl-D-tartaric (811 mg, 2.10 mmol) were dissolved in 50 mL of ethanol at reflux and stirred for 30 min. The resulting mixture was cooled down to room temperature and stirred for 3 hours. Then, the resulting solid was filtered off and washed with cold ethanol. Afterwards, the solid obtained was dried at 40°C under atmosphere pressure for 15 hours.
Yield: 1.3 g
Purity (HPLC): 99.9%
Enantiomeric purity (chiral HPLC method): 99.7%
IR (cm⁻¹): 2966, 2928, 2869, 1726, 1703, 1655, 1608, 1266, 1175, 1110, 841, 747. DSC: Sharp endotherm with an endothermic peak with a maximum at approximately 178 °C.

### Example 9. Comparative examples

Compounds of formula **IILHA,** wherein R₁ is benzyl, containing tartaric acid and derivates thereof and other organic acids were tested following the procedure of example 6. The following results obtained are gathered in table 1.

**Table 1.- Chemical characteristics of compounds of formula IILHA**

| **Compound of formula III.B (wherein R₁ is benzyl) of step ii)** | **Tartaric acid and derivates thereof** | **Yield of compound III.HA** | **Enantiomeric purity of compound III.HA** | **Purity (HPLC) of compound III.AHA** |
|---|---|---|---|---|
| Enantiomeric | L-tartaric | 75% | 99,42% | 99,39% |
| purity of 93.3% | ditoluyl-L-tartaric | 83% | 97,31% | 99,64% |
| and | ditoluyl-D-tartaric | 78% | 96,57% | 99,54% |
| chemical purity of 98,9% | D-tartaric | No solid was formed | - | - |
| | **Other organic acids** | **Yield** | **Enantiomeric purity** | **Purity (HPLC)** |
| | (1*R*)-(-)-10-camphorsulfonic | No solid was formed | - | - |
| | (*R*)-(-)-mandelic | No solid was formed | - | - |
| | maleic | 64% | 95,32% | 99,56% |
| | benzoic | 60% | 94,60% | 99,45% |

### Example 10. Preparation of (R)-5-[2-(5,6-diethyl-indan-2-ylamino-1-hydroxyethyl]-8-hydroxy-1H-quinolin-2-one (2Z)-2-butenedioate

(*R*)-5-[2-(5,6-diethyl-indan-2-ylamino-1-hydroxyethyl]-8-benzyloxy-1H-quinolin-2-one L-tartrate (2.0 g, 1.8 mmol) (enantiomeric purity: 99.87%; chemical purity: 99.62%) was dissolved in ethyl acetate (20 mL) and a solution of sodium hydroxide 1 M (15 mL) was added. The aqueous phase was extracted three times with 5 mL of ethyl acetate. Then, organic phases were combined, washed with 5 mL of water and the solvent was eliminated in vacuo. The resulting solid obtained (*R*)-8-(benzyloxy)--5-[2-(5,6-diethyl-indan-2-ylamino-1-hydroxyethyl]-1H-quinolin-2-one (enantiomeric purity: 99.71%; chemical purity: 98.44%) was dissolved in ethanol (20 mL) and mixed with 10% Pd/C (300 mg). The mixture was hydrogenated under 1 atm of H₂ at 50°C for 2 hours. The Pd/C was filtered through a Celite pad. The filtrate was evaporated and the residue was dissolved in ethanol (15 mL). The solution was heated to 60 °C and a solution of maleic acid (660 mg) in ethanol (1.5 mL) was added. The mixture was cooled down to room temperature and stirred for 3 hours. The suspension was filtered off and washed with cold ethanol. The white solid obtained was dried at 40 °C for 12 hours.
Yield: 0.8 g
Purity (HPLC): 99.7%
Enantiomeric purity (chiral HPLC method): 98.6%

(*R*)-5-[2-(5,6-diethyl-indan-2-ylamino-1-hydroxyethyl]-8-hydroxy-1 H-quinolin-2-one (2Z)-2-butenedioate was recrystallized in ethanol. Purity (HPLC): 99.9%. Enantiomeric purity (chiral HPLC method): 99.4%

## Claims

1. A process for manufacturing 5-[(R)-2-(5,6-diethyl-indan-2-ylamino)-1-hydroxy-ethyl]-8-hydroxy-(1H)-quinolin-2-one, indacaterol, of formula **I** or a pharmaceutically acceptable salt or co-crystal thereof, which comprises the steps of:
i) reacting compound of formula **II** 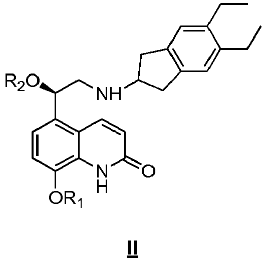 wherein R₁ is an aralkyl group and R₂ is a silyl group, with an acid, HX, in the presence of an organic solvent to provide a compound of formula **III**, 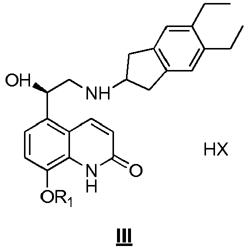
wherein R₁ is as defined above,
and wherein the acid, HX, is selected from the group consisting of hydrochloric acid, hydrobromic acid, phosphoric acid, sulfuric acid, formic acid, acetic acid, dichloroacetic acid, methansulfonic acid, *p*-toluensulfonic acid and camphorsulfonic acid,
ii) optionally converting the compound of formula **III** to a compound of formula **III.B** by treatment with a base in the presence of a solvent, wherein the solvent is a mixture of an organic solvent and water, 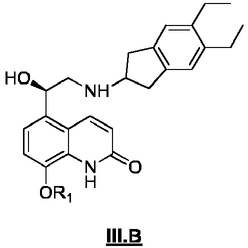
iii) removing the protecting group R₁ from the products resulting from step i) or ii), when present, by hydrogenation in the presence of a catalyst and an organic solvent to provide indacaterol of formula **I**, 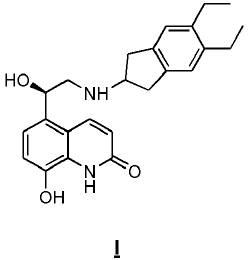
iv) optionally, treating the indacaterol of formula **I** obtained in step iii) with a pharmaceutically acceptable acid to provide a pharmaceutically acceptable salts or co-crystal thereof.

2. The process according to claim 1, wherein R₁ is a C₁-C₄alkyl group, substituted by a C₆-C₁₀ aryl group.

3. The process according to claim 2, wherein R₁ is benzyl or *p*-methoxybenzyl.

4. The process according to claim 3, wherein R₁ is benzyl.

5. The process according to any one of the preceding claims, wherein R₂ is selected from the group consisting of triethylsilyl and tert-butyldimethylsilyl.

6. The process according to any one of the preceding claims, wherein the acid, HX, is hydrochloric acid.

7. The process according to any one of the preceding claims, wherein the molar ratio of the compound of formula **II** to the acid HX is from 1:1 to 1:15.

8. The process according to any one of the preceding claims wherein the step ii) further comprises a purification treatment to increase the enantiomeric purity of compound III.B comprising the treatment of compound **III.B** with a chiral organic acid HA selected from the group consisting of L-tartaric acid, dibenzoyl-L-tartaric acid, dibenzoyl-D-tartaric, ditoluyl-L-tartaric acid and ditoluyl-D-tartaric in the presence of an organic solvent to provide a compound of formula **III.HA** 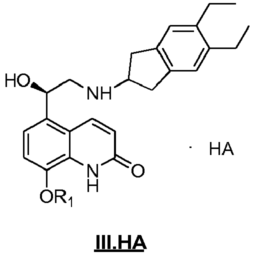 and, optionally converting compound of formula **III.HA** into compound of formula **III.B** by treatment with a base in the presence of a solvent, wherein the solvent is a mixture of organic solvent and water.

9. The process according to claim 8, wherein the chiral organic acid HA is L-tartaric acid.

10. The process according to any one of the preceding claims, wherein the compound of formula **III**, the compound of formula **III.B** and/or the compound of formula **III.HA** obtained in any of the steps i) or ii) are purified by means of conventional purification techniques.

11. The process according to any one of the preceding claims, wherein the organic solvent in steps i) and iii) is independently selected from the group consisting of alcohols, ethers and esters.

12. The process according to claim 11, wherein the organic solvent is an alcohol selected from the group consisting of methanol, ethanol, isopropanol and mixtures thereof.

13. The process according to any of the preceding claims, wherein the hydrogenation in step iii) is carried out in the presence of a base.

14. The process according to any of the preceding claims, wherein the pharmaceutically acceptable acid of step iv) is maleic acid to provide indacaterol maleate.

## Patentansprüche

1. Verfahren zur Herstellung von 5-[(R)-2-(5,6-Diethylindan-2-ylamino)-1-hydroxyethyl]-8-hydroxy-(1H)chinolin-2-on, Indacaterol, der Formel **I** oder eines pharmazeutisch annehmbaren Salzes oder Cokristalls davon, umfassend die Schritte:
i) Umsetzen einer Verbindung der Formel **II** 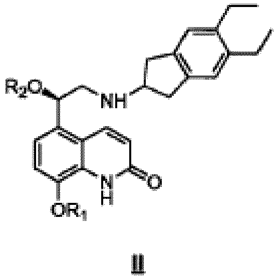 wobei R₁ eine Aralkylgruppe ist und R₂ eine Silylgruppe ist, mit einer Säure HX in Gegenwart eines organischen Lösungsmittels, so dass man eine Verbindung der Formel **III** erhält: 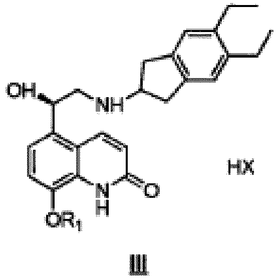
wobei R₁ wie oben definiert ist,
und wobei die Säure HX aus der Gruppe ausgewählt ist, die aus Chlorwasserstoffsäure, Bromwasserstoffsäure, Phosphorsäure, Schwefelsäure, Ameisensäure, Essigsäure, Dichloressigsäure, Methansulfonsäure, p-Toluolsulfonsäure und Kamphersulfonsäure besteht;
ii) gegebenenfalls Umsetzen der Verbindung der Formel **III** zu einer Verbindung der Formel **III.B** durch Behandlung mit einer Base in Gegenwart eines Lösungsmittels, wobei das Lösungsmittel ein Gemisch eines organischen Lösungsmittels mit Wasser ist: 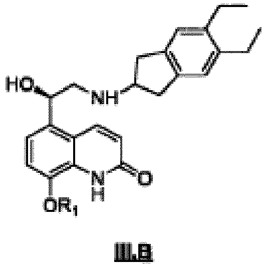
iii) Entfernen der Schutzgruppe R₁ von den Produkten, die aus Schritt i) oder ii) resultieren, falls vorhanden, durch Hydrierung in Gegenwart eines Katalysators und eines organischen Lösungsmittels, wobei man Indacaterol der Formel **I** erhält: 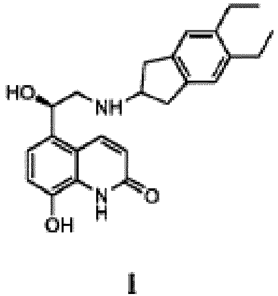
iv) gegebenenfalls Behandeln des in Schritt iii) erhaltenen Indacaterols der Formel **I** mit einer pharmazeutisch annehmbaren Säure, wobei man pharmazeutisch annehmbare Salze oder Cokristalle davon erhält.

2. Verfahren gemäß Anspruch 1, wobei R₁ eine C₁-C₄-Alkylgruppe ist, die mit einer C₆-C₁₀-Arylgruppe substituiert ist.

3. Verfahren gemäß Anspruch 2, wobei R₁ Benzyl oder p-Methoxybenzyl ist.

4. Verfahren gemäß Anspruch 3, wobei R₁ Benzyl ist.

5. Verfahren gemäß einem der vorstehenden Ansprüche, wobei R₂ aus der Gruppe ausgewählt ist, die aus Triethylsilyl und tert-Butyldimethylsilyl besteht.

6. Verfahren gemäß einem der vorstehenden Ansprüche, wobei es sich bei der Säure HX um Chlorwasserstoffsäure handelt.

7. Verfahren gemäß einem der vorstehenden Ansprüche, wobei das Stoffmengenverhältnis der Verbindung der Formel **II** zu der Säure HX 1:1 bis 1:15 beträgt.

8. Verfahren gemäß einem der vorstehenden Ansprüche, wobei Schritt ii) weiterhin eine Reinigungsbehandlung umfasst, um die Enantiomerenreinheit von Verbindung III.B zu erhöhen, umfassend die Behandlung von Verbindung **III.B** mit einer chiralen organischen Säure HA, die aus der Gruppe ausgewählt ist, die aus L-Weinsäure, Dibenzoyl-L-weinsäure, Dibenzoyl-D-weinsäure, Ditoluyl-L-weinsäure und Ditoluyl-D-weinsäure besteht, in Gegenwart eines organischen Lösungsmittels, wobei man eine Verbindung der Formel **III.HA** erhält: 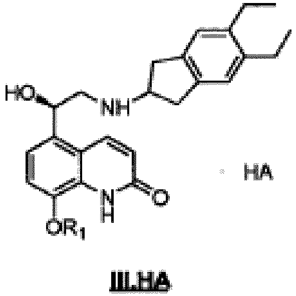 und gegebenenfalls Umsetzen der Verbindung der Formel **III.HA** zu einer Verbindung der Formel **III.B** durch Behandlung mit einer Base in Gegenwart eines Lösungsmittels, wobei das Lösungsmittel ein Gemisch eines organischen Lösungsmittels mit Wasser ist.

9. Verfahren gemäß Anspruch 8, wobei es sich bei der chiralen organischen Säure HA um L-Weinsäure handelt.

10. Verfahren gemäß einem der vorstehenden Ansprüche, wobei die Verbindung der Formel **III,** die Verbindung der Formel **III.B** und/oder die Verbindung der Formel **III.HA,** die in einem der Schritte i) oder ii) erhalten werden, mittels herkömmlicher Reinigungstechniken gereinigt werden.

11. Verfahren gemäß einem der vorstehenden Ansprüche, wobei das organische Lösungsmittel in den Schritten i) und iii) unabhängig aus der Gruppe ausgewählt ist, die aus Alkoholen, Ethern und Estern besteht.

12. Verfahren gemäß Anspruch 11, wobei das organische Lösungsmittel ein Alkohol ist, der aus der Gruppe ausgewählt ist, die aus Methanol, Ethanol, Isopropanol und Gemischen davon besteht.

13. Verfahren gemäß einem der vorstehenden Ansprüche, wobei die Hydrierung in Schritt iii) in Gegenwart einer Base durchgeführt wird.

14. Verfahren gemäß einem der vorstehenden Ansprüche, wobei es sich bei der pharmazeutisch annehmbaren Säure von Schritt iv) um Maleinsäure handelt, so dass man Indacaterolmaleat erhält.

## Revendications

1. Un procédé de fabrication de 5-[(R)-2-(5,6-diéthyl-indan-2-ylamino)-1-hydroxy-éthyl]-8-hydroxy-(1H)-quinolin-2-one, indacatérol, de la formule I ou d'un sel ou co-cristal pharmaceutiquement acceptable de celui-ci, qui comprend les étapes suivantes :
i) faire réagir le composé de formule **II** 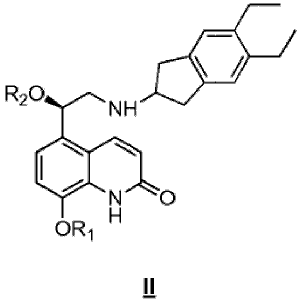 où R₁ est un groupe aralkyle et R₂ est un groupe silyle, avec un acide, HX, en présence d'un solvant organique pour obtenir un composé de formule **III,** 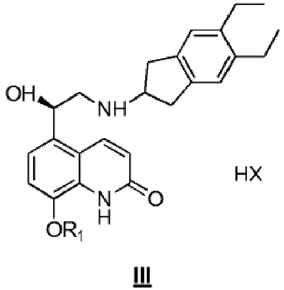
où R₁ est tel que défini ci-dessus,
et où l'acide HX est sélectionné parmi le groupe constitué d'acide chlorhydrique, acide bromhydrique, acide phosphorique, acide sulfurique, acide formique, acide acétique, acide dichloroacétique, acide méthanesulfonique, acide p-toluènesulfonique et acide camphorsulfonique,
ii) éventuellement convertir le composé de formule **III** en un composé de formule **III.B** par traitement à l'aide d'une base en présence d'un solvant, où le solvant est un mélange d'un solvant organique et d'eau, 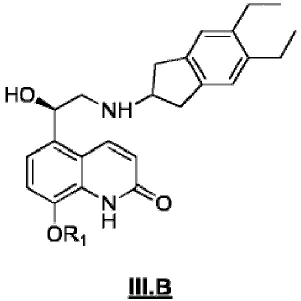
iii) éliminer le groupe protecteur R₁ des produits obtenus à l'étape i) ou ii), si présent, par hydrogénation en présence d'un catalyseur et d'un solvant organique afin d'obtenir l'indacatérol de formule **I**, 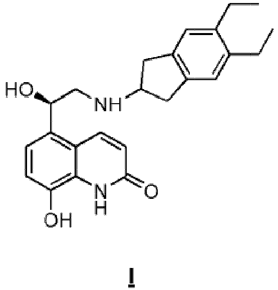
iv) éventuellement, traiter l'indacatérol de formule **I** obtenu à l'étape iii) à l'aide d'un acide pharmaceutiquement acceptable afin d'obtenir un sel ou co-cristal pharmaceutiquement acceptable de celui-ci.

2. Le procédé selon la revendication 1, où R₁ est un groupe alkyle en C₁-C₄, substitué par un groupe aryle en C₆-C₁₀.

3. Le procédé selon la revendication 2, où R₁ est un benzyle ou un p-méthoxybenzyle.

4. Le procédé selon la revendication 3, où R₁ est un benzyle.

5. Le procédé selon l'une quelconque des revendications précédentes, où R₂ est sélectionné parmi le groupe constitué de triéthylsilyle et tert-butyldiméthylsilyle.

6. Le procédé selon l'une quelconque des revendications précédentes, où l'acide HX est de l'acide chlorhydrique.

7. Le procédé selon l'une quelconque des revendications précédentes, où le rapport molaire du composé de formule **II** à l'acide HX est de 1:1 à 1:15.

8. Le procédé selon l'une quelconque des revendications précédentes où l'étape ii) comprend en outre un traitement de purification afin d'accroître la pureté énantiomérique du composé III.B comprenant le traitement du composé **III.B** à l'aide d'un acide organique chiral HA sélectionné parmi le groupe constitué d'acide L-tartrique, acide dibenzoyle-L-tartrique, dibenzoyle-D-tartrique, acide ditoluyl-L tartrique et ditoluyl-D-tartrique en présence d'un solvant organique afin d'obtenir un composé de formule **III.HA** 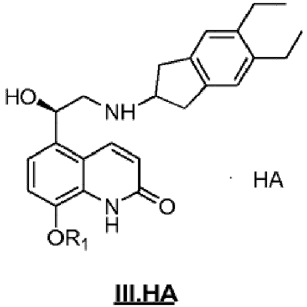 et, éventuellement convertir le composé de formule **III.HA** en un composé de formule **III.B** par traitement à l'aide d'une base en présence d'un solvant, où le solvant est un mélange de solvant organique et d'eau.

9. Le procédé selon la revendication 8, ou l'acide organique chiral HA est de l'acide L-tartrique.

10. Le procédé selon l'une quelconque des revendications précédentes, où le composé de formule **III**, le composé de formule **III.B** et/ou le composé de formule **III.HA** obtenus dans l'une quelconque des étapes i) ou ii) sont purifiés à l'aide de techniques classiques de purification.

11. Le procédé selon l'une quelconque des revendications précédentes, où le solvant organique aux étapes i) et iii) est indépendamment sélectionné parmi le groupe constitué d'alcools, d'éthers et d'esters.

12. Le procédé selon la revendication 11, où le solvant organique est un alcool sélectionné parmi le groupe constitué de méthanol, éthanol, isopropanol et de mélanges de ceux-ci.

13. Le procédé selon l'une quelconque des revendications précédentes, où l'hydrogénation à l'étape iii) est réalisée en présence d'une base.

14. Le procédé selon l'une quelconque des revendications précédentes, où l'acide pharmaceutiquement acceptable de l'étape iv) est de l'acide maléique afin d'obtenir du maléate d'indacatérol.
